# EUROPÄISCHE PATENTSCHRIFT

(11) **EP 4 531 767 B1**
(45) Veröffentlichungstag und Bekanntmachung des Hinweises auf die Patenterteilung: **03.06.2026**
(21) Anmeldenummer: 23732364.7
(22) Anmeldetag: 19.05.2023
(51) Int. Cl.: A61F 5/01

(54) **EINSTELLBARES ORTHESENGELENK ZUR KONTROLLIERTEN BEWEGUNG UND/ODER FIXIERUNG EINER HAND SOWIE ORTHESE MIT EINEM DERARTIGEN ORTHESENGELENK**
ADJUSTABLE ORTHOSIS JOINT FOR THE CONTROLLED MOVEMENT AND/OR FIXATION OF A HAND, AND ORTHOSIS COMPRISING SUCH AN ORTHOSIS JOINT
ARTICULATION D'ORTHÈSE RÉGLABLE POUR LA FIXATION ET/OU LE MOUVEMENT CONTRÔLÉS D'UNE MAIN, ET ORTHÈSE COMPRENANT UNE TELLE ARTICULATION D'ORTHÈSE

(30) Priorität: 02.06.2022 DE 102022114050; 01.09.2022 DE 102022122114
(43) Veröffentlichungstag der Anmeldung: 09.04.2025
(73) Patentinhaber: Kimebo UG (Haftungsbeschränkt), 80331 München (DE)
(72) Erfinder: KLIMMER, Uwe, 80331 München (DE); GANSER, Klaus, 82205 Gilching (DE); DIEBL, Norbert, 82327 Tutzing / Traubing (DE)
(74) Vertreter: Heilein, Ernst-Peter
(86) Internationale Anmeldenummer: PCT/DE2023/100370
(87) Internationale Veröffentlichungsnummer: WO 2023/232185

(56) Entgegenhaltungen:
- WO-A1-2023/025354
- WO-A2-2021/093919
- US-A- 5 520 625

## Beschreibung

Die vorliegende Erfindung betrifft ein einstellbares Orthesengelenk zur kontrollierten Bewegung und/oder Fixierung einer Hand eines Patienten, wenigstens umfassend ein Bewegungsmodul zur Kontrolle einer Extensions- und/oder Flexionsbewegung sowie zur Kontrolle und Ausführung einer ulnaren und/oder radialen Deviation der Hand; und einen, mit dem Bewegungsmodul wirkverbundenen Schwenkhebel zur Ausführung der Extensions- und/oder Flexionsbewegung der Hand; sowie eine Orthese zur kontrollierten Bewegung und/oder Fixierung einer Hand oder einer Hand und eines Unterarms eines Patienten mit einem derartigen Orthesengelenk.

Im Rahmen des Rehabilitationsprozesses nach einer Handverletzung muss die betroffene Hand bzw. der Unterarm des Patienten in vielen Fällen eine gewisse Zeit lang ruhiggestellt werden. Die Ruhigstellung erfolgt dabei gewöhnlich mit Hand-/Unterarm-Orthesen, welche Hand und Unterarm in einer bestimmten Position fixieren und nach erfolgter Fixierung keinerlei Bewegung der besagten Gliedmaßen mehr zulassen. Dabei werden allerdings auch zwangsläufig Gelenke der Hand und/oder des Unterarms ruhiggestellt, die gar nicht von der Verletzung betroffen sind. Je nach Tragedauer einer solchen Orthese kann dies nachteilig zu sog. Sekundär-Versteifungen der von der Verletzung nicht betroffenen Gelenke führen, deren zusätzliche Mobilisation nach Ende des Heilungsverlaufs unnötige Kosten wegen einer verlängerten Rehabilitationszeit und somit längerer Arbeitsausfallzeiten des Patienten verursacht. Bei komplexen Verletzungen der Hand bzw. des Unterarms kann es zudem wünschenswert sein, einzelne, in ihrem Heilungsprozess weiter fortgeschrittene Gelenke bereits kontrolliert zu bewegen und dabei gleichzeitig noch stärker geschädigte Gelenke weiterhin zu fixieren. Unter einer "kontrollierten Bewegung" soll in diesem Zusammenhang insbesondere eine, in ihrem Bewegungswinkel eingeschränkt, geführte Bewegung verstanden werden. In der Physiotherapie und Orthopädie werden hierzu unter anderem oftmals Orthesen eingesetzt, welche Gliedmaßen aufnehmen und die Bewegung eines bestimmten Gelenks in einem vorgegebenen Winkelbereich freigeben, so dass der Patient die Bewegung selbstständig und sicher ausführen kann. Der Winkelbereich kann dann, je nach dem Trainings- bzw. Gesundheitszustand des Patienten, nach und nach erweitert werden, um schließlich den ursprünglichen Bewegungsradius wiederzuerlangen.

Aus dem Stand der Technik sind solche "range-of-motion" (ROM)-Vorrichtungen insbesondere für Knie-Orthesen bzw. für die Beuge- und Streckbewegung des Knies bekannt. Die EP 0 546 331 A1 und die EP 0 832 624 A2 offenbaren beispielsweise Orthesengelenke mit einstellbarem Bewegungsumfang bezogen auf eine Bewegung in einer Ebene, welche vorzugsweise in einer Knie-Orthese zum Einsatz kommen können. Aus der US 7,081,102 B1 ist zudem eine Karpaltunnel-Stütze mit Gelenk bekannt, welche eine eingeschränkte, radiale und/oder ulnare Deviation der Hand des Benutzers erlaubt.

Das menschliche Handgelenk zeichnet sich allerdings durch einen komplexeren, "mehrdimensionalen" Bewegungsumfang aus, welcher durch eine Kombination aus einer Extensions-/Flexions-Bewegung (Beuge- und Streckbewegung in einer Ebene senkrecht zur Handfläche) und aus einer ulnaren bzw. radialen Deviation (Wischbewegung in der Ebene der Handfläche) beschrieben werden kann. Die aus dem Stand der Technik bekannten, in ihrem Bewegungsumfang einstellbaren Orthesengelenke und ROM-Vorrichtungen lassen die Kontrolle einer derartigen mehrdimensionalen Bewegung nicht zu bzw. benötigen mehrere, getrennte Gelenke. Exemplarisch seien hierzu die WO 2021/093 919 A2, die US 2021/014 56 20 A1, die US 5 520 625 A und die CN 210 843 695 U genannt. In diesem Zusammenhang sei auch auf die DE 10 2019 130 404 A1 der Anmelderin hingewiesen, welche eine Orthese zur kontrollierten Bewegung und/oder Fixierung eines Unterarms und/oder einer Hand beschreibt, die der beschriebenen mehrdimensionalen Bewegung Rechnung trägt, hierzu jedoch nachteilig baulich vergleichsweise ausladend ausgestaltet ist und einen Alltagsgebrauch der Orthese durch Benutzer praktisch verhindert.

Orthesengelenke des Stands der Technik, wie beispielsweise aus der WO 03/070129 A1 bekannt, ermöglichen zwar in kompakterer Bauform die Kontrolle eines einzigen Bewegungsfreiheitsgrades, vermögen es jedoch nicht, die beschriebene, mehrdimensionale Bewegung des Handgelenks abzubilden.

Zur Lösung dieses Problems wird mit der DE 10 2021 121 819.5 der Anmelderin bereits ein einstellbares Orthesengelenk bereitgestellt, welches technisch der komplexen Kombination aus einer Extensions-/Flexions-Bewegung und einer ulnaren bzw. radialen Deviation Rechnung trägt. Das darin beschriebene Orthesengelenk ist besonders stabil ausgelegt und ermöglicht vorteilhaft eine Benutzung während vergleichsweise schwerer Berufs- und Alltagstätigkeiten. Allerdings bedingt besagte Robustheit auch eine gewisse Bauform, welche einerseits nur unter Zuhilfenahme von Werkzeug auf die individuellen Bedürfnisse einzelner Patienten eingestellt werden kann und welche bestimmten Personengruppen, die nur vergleichsweise leichte und mittelschwere Tätigkeit verrichten müssen, bei längerer Benutzung als zu groß und dadurch störend empfinden.

Hiervon ausgehend liegt der vorliegenden Erfindung die Aufgabe zugrunde, ein alternatives Orthesengelenk, insbesondere für eine Hand-Orthese, bereitzustellen, welches den bestehende Stand der Technik dahingehend verbessert ist, dass es die Kontrolle sowohl einer Extensions-/Flexionsbewegung als auch einer ulnaren und/oder radialen Deviation der Hand eines Patienten ermöglicht und gleichzeitig so benutzerfreundlich ausgestaltet ist, dass es ohne Verwendung von speziellem Werkzeug vom Patienten selbst auf die sich ändernden individuellen Bedürfnisse während der fortschreitenden Rehabilitation eingestellt werden kann und gleichzeitig so kompakt gebaut ist, dass seine Benutzung vom Patienten im Alltag als angenehm bzw. nicht störend empfunden wird.

Diese Aufgabe wird durch ein einstellbares Orthesengelenk mit den Merkmalen des unabhängigen Patentanspruchs 1 bzw. durch eine Orthese mit den Merkmalen des nebengeordneten Patentanspruchs 14 gelöst.

Das erfindungsgemäße Orthesengelenk zeichnet sich gegenüber Orthesengelenken des Stands der Technik dadurch aus, dass das Bewegungsmodul wenigstens ein Grundelement und wenigstens ein Kontrollelement mit einer Oberseite und einer dem Grundelement zugewandten und der Oberseite gegenüberliegenden Unterseite umfasst,
- wobei an der Unterseite ein Drehzylinder angeordnet ist, mittels dessen das Kontrollelement gegenüber dem Grundelement bezüglich einer Deviationsachse drehbar gelagert ist,
- wobei an der Unterseite eine erste Aussparung zur Deviationskontrolle angeordnet ist;
- wobei das Kontrollelement wenigstens einen ersten Kontrollschieber umfasst, welcher mittels wenigstens eines ersten Stellmittels bezüglich des mit dem Bewegungsmodul wirkverbundenen Schwenkhebels verschiebbar ist;
- und wobei das Kontrollelement eingerichtet ist; die Bewegung des Schwenkhebels in seinem Bewegungsumfang mittels des ersten Kontrollschiebers kontrolliert zu beschränken;
- und dass das Orthesengelenk wenigstens einen, mit dem Kontrollelement zusammenwirkenden zweiten Kontrollschieber für die Kontrolle der ulnaren/radialen Deviationsbewegung der Hand umfasst;
- wobei das Kontrollelement eingerichtet ist, in seiner Drehbewegung um die Deviationsachse durch ein Zusammenwirken der ersten Aussparung zur Deviationskontrolle und des wenigstens einen zweiten Kontrollschiebers kontrolliert beschränkt zu werden.

Mit Hilfe des ersten Kontrollschiebers kann vorteilhaft zur Kontrolle der Extensions- und Flexionsbewegung der Hand, die Wirkverbindung zwischen Bewegungsmodul und Schwenkhebel sicher und einfach verändert und so auf die individuellen Bedürfnisse des Patienten eingestellt werden

Das erfindungsgemäße einstellbare Orthesengelenk ermöglicht es vorteilhaft, die Winkel der Extension und Flexion sowie der ulnaren und radialen Deviation der Hand je nach Gesundheitszustand und Therapiebedarf des Patienten zu kontrollieren. Der Winkel der Extension bzw. Flexion kann dabei vorteilhaft entweder bei 180° (|W_{E/F}| = 0°; ausgestreckte Hand) fixiert oder in einem Bewegungsbereich von 180° ± |W_{E/F}| mit |W_{E/F}| > 0° kontrolliert freigegeben werden. Der Winkelbereich von 180° bis 180° - |W_{E/F}| entspricht hierbei einer Dorsalextension, der Winkelbereich von 180° bis 180° + |W_{E/F}| einer Palmarflexion. Die Winkel der ulnaren und radial Deviation, auch als "Ulnarabduktion" und "Radialabduktion" bezeichnet, können entsprechend vorteilhaft entweder bei |W_{D1}| = |W_{D2}| = 0° (Hand zeigt gerade nach vorne) fixiert oder in einem Bewegungsbereich von |W_{D1}|, |W_{D2}| > 0° kontrolliert freigegeben werden. Die Beträge der Winkel |W_{D1}| und |W_{D2}| können dabei gleich groß aber auch unterschiedlich groß sein, so dass ein unsymmetrischer Bewegungsrahmen vorgegeben werden kann. Die Bewegungsachsen für die Extensions- und Flexionsbewegung bzw. für die ulnare und radiale Deviation, die vom Bewegungsmodul des erfindungsgemäßen Orthesengelenk technisch realisiert werden, liegen dabei vorteilhaft im Bereich der physiologischen Gelenkachsen und bilden auf diese Weise den "natürlichen" Bewegungsumfang des Handgelenks bzw. der Hand besonders gut ab.

Eine erfindungsgemäße Orthese mit einem solchen einstellbaren Orthesengelenk kann den Heilungsverlauf vorteilhaft in der Weise begleiten, dass die Bewegungsfreiheit des Patienten kontrolliert erhöht werden kann, wobei aus therapeutischer Sicht zugelassene Bewegungen kontrolliert geführt werden und gleichzeitig aus therapeutischer Sicht kritische Bewegungen vollständig oder teilweise eingeschränkt werden können. Auf diese Weise kann einer unnötigen Versteifung gesunder Gelenke der Hand vorteilhaft entgegengewirkt werden. Das erfindungsgemäße Orthesengelenk weist dabei eine besonders kompakte Bauweise auf, sodass eine damit ausgestattete erfindungsgemäße Orthese vorteilhaft ebenfalls kompakt ausgestaltet sein kann und den Patienten in seinem Alltag wenig behindert.

Weitere vorteilhafte Ausgestaltungen und Weiterbildungen, welche einzeln oder in Kombination miteinander einsetzbar sind, sind Gegenstand der abhängigen Ansprüche.

In einer Ausgestaltung der Erfindung hat sich bewährt, wenn der erste Kontrollschieber im Wesentlichen quaderförmig ausgestaltet und an seinem dem Schwenkhebel zugewandten Ende gabelförmig ausgebildet ist. Ein verschiebbarer, quaderförmiger erster Kontrollschieber mit gabelförmigem Ende kann vorteilhaft so an den Schwenkhebel herangeschoben werden, dass insbesondere das mittels einer Schwenkachse schwenkbar gelagerte Ende des Schwenkhebels von den "Gabelzinken" eingefasst wird, das Ende des Schwenkhebels sich also im Innenraum des gabelförmigen Endes des ersten Kontrollschiebers befindet. Die "Gabelzinken", also der obere und der untere Teil des gabelförmigen Endes, können dabei vorteilhaft jeweils als Anschlag fungieren und das Ausmaß der Schwenkbewegung des Schwenkhebels begrenzen.

In einer weiteren bevorzugten Ausgestaltung der Erfindung hat sich bewährt, wenn das erste Stellmittel wenigstens einen Kopf und einen Stellzylinder umfasst, welche, vorzugsweise über einen Kragen, miteinander verbunden sind, sodass sich ein Bauteil mit einer Längsachse bildet, wobei der Stellzylinder einen kreisförmigen Querschnitt aufweist und bezüglich der Längsachse außermittig mit dem Kopf des ersten Stellmittels verbunden ist. Der erste Kontrollschieber kann dann vorteilhaft wenigstens eine Aussparung umfassen, welche eingerichtet ist, den Stellzylinder aufzunehmen und mit diesem wechselzuwirken. Ein erstes Stellmittel mit einem Stellzylinder, welcher einen kreisförmigen Querschnitt aufweist und außermittig mit dem Kopf verbunden ist, kann, wenn das erste Stellmittel durch die Aussparung des ersten Kontrollschiebers aufgenommen ist, diesen dadurch verschieben, dass der Stellzylinder beim Drehen des ersten Stellmittels mit einer Seitenbegrenzung der Aussparung in Wirkverbindung gelangt, Kraft auf besagte Seitenbegrenzung ausübt und dadurch den ersten Kontrollschieber von der Rotationsachse des ersten Stellmittels wegschiebt. Aufgrund der außermittigen Anordnung des Stellzylinders im Sinne einer Exzenterschraube verringert sich, wenn das erste Stellmittel in die Gegenrichtung gedreht wird, die Wirkverbindung mit besagter Seitenbegrenzung und vergrößert sich mit einer gegenüberliegenden Seitenbegrenzung der Aussparung, sodass der erste Kontrollschieber entsprechend in die andere Richtung geschoben wird.

Alternativ dazu hat sich eine Ausgestaltung eines Orthesengelenks bewährt, bei der das erste Stellmittel wenigstens einen Kopf und einen Stellzylinder umfasst, welche, vorzugsweise über einen Kragen, miteinander verbunden sind, sodass sich ein Bauteil mit einer Längsachse bildet, wobei der Stellzylinder an seiner dem Kopf abgewandten Seite einen, vorzugsweise im Wesentlichen C-förmigen, Kanal aufweist. Der erste Kontrollschieber kann dann entsprechend einen Verbindungsstift umfassen, welcher bei Gebrauch des Orthesengelenks in den Kanal des Stellzylinders eingreift, wobei der Verbindungsstift vorzugsweise innerhalb einer Aussparung des ersten Kontrollschiebers angeordnet ist, welche eingerichtet ist, den Stellzylinder aufzunehmen. Bei einer Drehung eines derart ausgestalteten ersten Stellmittels kann über den Verbindungsstift, welcher in den im Wesentlichen C-förmigen Kanal des Stellzylinders eingreift, durch Wechselwirkung mit den Wänden des Kanals vorteilhaft Kraft auf den ersten Kontrollschieber ausgeübt werden. Je nach Drehrichtung kann der erste Kontrollschieber dadurch vorteilhaft in Richtung des Schwenkhebels oder in Gegenrichtung verschoben werden.

Darüber hinaus hat sich eine Ausgestaltung bewährt, bei der das erste Stellmittel und/oder der erste Kontrollschieber wenigstens einen Einrastmechanismus umfassen, welcher eingerichtet ist, den ersten Kontrollschieber in seiner Verschiebbewegung bezüglich des mit dem Bewegungsmodul wirkverbundenen Schwenkhebels an wenigstens einer vorgegebenen Position reversibel zu fixieren. Ein Einrastmechanismus ermöglicht vorteilhaft vorgegebene maximale mögliche Extensions-/Flexionswinkel sicher und schnell einzustellen.

In einer weiteren bevorzugten Ausgestaltung der Erfindung kann sich die erste Aussparung zur Deviationskontrolle von einem Rand des Kontrollelements in Richtung des Drehzylinders erstrecken, sodass eine Höhe des Kontrollelements im Bereich der ersten Aussparung kleiner ist als eine Höhe des Kontrollelements im Bereich außerhalb der ersten Aussparung. Dadurch können sich wenigstens vier Seitenkanten bilden, die den Bereich innerhalb der ersten Aussparung vom Bereich außerhalb der ersten Aussparung abgrenzen, von denen je zwei in einem Abstand dₙ mit n = 1, 2, ... einander im Wesentlichen gegenüberliegen und ein Seitenkantenpaar SPₙ mit n = 1, 2, ... bilden; wobei ein Abstand dₙ zwischen zwei näher am Drehzylinder angeordneten, einander gegenüberliegenden Seitenkanten eines Seitenkantenpaares SPₙ stets kleiner ist als ein Abstand dₙ₊₁ zwischen zwei näher am Rand des Kontrollelements angeordneten, einander gegenüberliegenden Seitenkanten eines Seitenkantenpaares SPₙ₊₁. Eine derart ausgestaltetes Kontrollelement ermöglicht vorteilhaft die Einstellung und Kontrolle von wenigstens zwei verschiedenen maximalen Drehwinkeln um die Deviationsachse, also eine Kontrolle der Winkel der ulnaren und radial Deviation. Besonders bevorzugt ist dabei eine Ausgestaltung der Erfindung, bei der eine Vielzahl von Seitenkanten, vorzugsweise acht Seitenkanten, gebildet werden, von denen je zwei einander im Wesentlichen gegenüber liegen und ein Seitenkantenpaar SPₙ mit n = 1, 2, ... bilden. Je mehr Seitenkantenpaare SPₙ vorgesehen werden, desto kleinschrittiger können die Winkelbereiche der ulnaren und radialen Deviation durch den Patienten und/oder Therapeuten eingeteilt werden. Das Vorsehen von vier Seitenkantenpaare SPₙ (entsprechend acht Seitenkanten) zur Einstellung von maximalen ulnaren/radialen Deviationsbewegungen von |W_{D1}| und |W_{D2}| = 0°, 10°, 20°, 30° hat sich dabei zur Erzeugung eines möglichst schnellen Rehabilitationsfortschritts als besonders geeignet erwiesen, es können jedoch auch beliebige andere Winkel vorgesehen werden.

Zudem hat sich eine Ausgestaltung bewährt, bei der der zweite Kontrollschieber im Wesentlichen quaderförmig ausgebildet ist und eine Länge, eine Breite und eine Höhe aufweist. Die Breite kann dabei vorzugsweise dem Abstand d₁ der einander gegenüberliegenden Seitenkanten des ersten, am nächsten zum Drehzylinder angeordneten Seitenkantenpaares SP₁ entsprechen; und der zweite Kontrollschieber kann in die erste Aussparung des Kontrollelements hineinführbar und herausführbar ausgestaltet sein und mit den Seitenkanten wechselwirken. Ein zweiter Kontrollschieber mit einer Breite, die dem Abstand d₁ der einander gegenüberliegenden Seitenkanten des ersten, am nächsten zum Drehzylinder angeordneten Seitenkantenpaares SP₁ entspricht, ermöglicht vorteilhaft, eine vollständige Fixierung der Hand in gerader Position bezüglich einer Deviationsbewegung, also in einer Position mit |W_{D1}| und |W_{D2}| = 0°, insbesondere dann, wenn der zweite Kontrollschieber vollständig an den Drehzylinder herangeschoben worden ist.

Alternativ dazu hat sich eine weitere Ausgestaltung des erfindungsgemäßen Orthesengelenks bewährt, bei dem zwei, mit dem Kontrollelement zusammenwirkende zweite Kontrollschieber für die Kontrolle der ulnaren/radialen Deviationsbewegung der Hand vorgesehen sind, wobei die zweiten Kontrollschieberjeweils im Wesentlichen quaderförmig ausgebildet sind und eine Länge, eine Breite und eine Höhe aufweisen; wobei die Summe der Breiten dem Abstand d₁ der einander gegenüberliegenden Seitenkanten des ersten, am nächsten zum Drehzylinder angeordneten Seitenkantenpaares SP1 entspricht. Ein jeder zweite Kontrollschieber kann dabei vorzugsweise in die erste Aussparung des Kontrollelements hineinführbar und herausführbar ausgestaltet sein und mit den Seitenkanten wechselwirken. Zwei zweite Kontrollschieber, deren Breiten addiert dem Abstand d₁ der einander gegenüberliegenden Seitenkanten des ersten, am nächsten zum Drehzylinder angeordneten Seitenkantenpaares SP₁ entsprechen, ermöglichen es vorteilhaft, den Gesamtbewegungsumfang der Deviationsbewegung der Hand in einen Winkelbereich der Ulnarabduktion (entsprechend |W_{D2}| > 0°) und in einen Winkelbereich der Radialabduktion (entsprechend |W_{D1}| > 0°) aufzuteilen und unabhängig voneinander zu kontrollieren. Dabei können vorteilhaft sowohl Bewegungen in einem Winkelbereich von 0° ≤ |W_{D2}| ≤ 30 ° für eine Fixierung bzw. kontrollierte Ulnarabduktion, Bewegungen in einem Winkelbereich von 0° ≤ |W_{D1}| ≤ 30 ° für eine Fixierung bzw. kontrollierte Radialabduktion, als auch eine Kombination verschiedener Winkel |W_{D1}|, |W_{D2}| vom Patienten oder Therapeuten eingestellt werden.

Darüber hinaus hat es sich in einer weiteren bevorzugten Ausgestaltung bewährt, wenn ein jeder zweite Kontrollschieber eingerichtet ist, an vorgegebenen Positionen während des Hineinführens in und Herausführens aus der ersten Aussparung reversibel fixiert zu werden, vorzugsweise mittels eines Einrastmechanismus. Dazu kann ein jeder zweite Kontrollschieber insbesondere an seiner zur Orthese hin gerichteten Seite ein Sägezahnprofil aufweisen, welches mit einer korrespondierend ausgestalteten, zum zweiten Kontrollschieber hin gerichteten Seite der Orthese und/oder eines Gehäuses für den oder die zweiten Kontrollschieber reversibel in Wirkverbindung treten und so einen Einrastmechanismus bilden kann. Alternativ oder kumulativ kann der Einrastmechanismus auch durch einen oder mehrere, vorzugsweise gefederte, Stifte oder Noppen gebildet werden, welche an der zum zweiten Kontrollschieber hin gerichteten Seite der Orthese und/oder des Gehäuses für den zweiten Kontrollschieber angeordnet sein können und mit korrespondierend ausgebildeten Vertiefungen, insbesondere Löchern, in der zur Orthese hin gerichteten Seite des oder der zweiten Kontrollschieber wechselwirken. Ein oder zwei derart eingerichtete Kontrollschieber ermöglichen es vorteilhaft, vorgegebene Deviationswinkel für eine maximal mögliche Ulnar- und/oder Radialabduktion sicher, schnell und ohne Verwendung von Werkzeug einzustellen.

In einer weiteren alternativen Ausgestaltung des erfindungsgemäßen Orthesengelenks können auch zwei, mit dem Kontrollelement zusammenwirkende zweite Kontrollschieber für die Kontrolle der ulnaren/radialen Deviationsbewegung der Hand und wenigstens eine, am Kontrollelement angeordnete, vierte Aussparung zur Deviationsfixierung vorgesehen sein. In dieser besonders bevorzugten Ausgestaltung kann dabei einer der zweiten Kontrollschieber als Deviationskontrollschieber ausgebildet sein und wenigstens einen Kopfbereich, welcher eingerichtet ist in die erste Aussparung des Kontrollelements hineingeführt und herausgeführt zu werden und mit den Seitenkanten der ersten Aussparung des Kontrollelements wechselzuwirken, und einen Körperbereich, welcher eine Führungsbahn für einen Kontrollstift eines, vom Orthesengelenk umfassten, zweiten Stellmittels aufweist, umfassen. Der andere der zwei zweiten Kontrollschieber kann dagegen als ein Fixierschieber ausgebildet sein und wenigstens einen Kopfbereich, welcher eingerichtet ist mit der vierten Aussparung des Kontrollelement wechselzuwirken, und einen Körperbereich, welcher eine Führungsbahn für den Kontrollstift des zweiten Stellmittels aufweist, umfassen. Der Deviationskontrollschieber und der Fixierschieber sind in dieser Ausgestaltung zudem vorzugsweise verschiebbar gelagert und so ausgebildet, dass sie sich mittels des Kontrollstifts des zweiten Stellmittels gegeneinander verschieben lassen.

Zwei derartig ausgebildete zweite Kontrollschieber, also ein als Deviationsschieber und ein als Fixierschieber ausgebildeter zweiter Kontrollschieber, welche mittels eines zweiten Stellmittels gegeneinander verschiebbar sind, ermöglichen vorteilhaft sowohl die Kontrolle der ulnare und radialen Deviation der Hand als auch eine Fixierung der Deviationsbewegung bei einem bestimmten Winkel mittels eines einzigen weiteren Bauteils, nämlich mittels des zweiten Stellmittels. Die Einstellung und Fixierung der ulnaren und radialen Deviation kann in dieser Ausgestaltung vorteilhaft über die Drehung des zweiten Stellmittels also, ähnlich einfach wie die Einstellung und Fixierung der Extensions- und Flexionsbewegung der Hand über Drehung des ersten Stellmittels, ohne Verwendung von weiterem, besonderen Werkzeug erfolgen.

Darüber hinaus hat sich bewährt, wenn dabei der Kopfbereich des Deviationskontrollschiebers von zwei, einander gegenüberliegenden Hebelelementen gebildet wird, welche gegeneinander federnd bewegbar sind und deren Außenkanten in einem entspannten Zustand zueinander einen Abstand aufweisen, der dem Abstand d₁ der einander gegenüberliegenden Seitenkanten des ersten, am nächsten zum Drehzylinder angeordneten Seitenkantenpaares SP₁ der ersten Aussparung entspricht. Der Kopfbereich des Fixierschiebers kann zudem vorzugsweise ein Kopfelement zur Wechselwirkung mit der vierten Aussparung umfassen, welches fest mit dem Körperbereich des Fixierschiebers verbunden ist. Der Kopfbereich eines derartig ausgebildeten Deviationskontrollschiebers kann vorteilhaft die Wechselwirkung der Außenkanten des Deviationskontrollschiebers mit den Seitenkanten der ersten Aussparung vorteilhaft dämpfen und dem Benutzer so das Gefühl eines weicheren Anschlags in die jeweilige Endposition der Deviationsbewegung vermitteln. Der Kopfbereich eines derart ausgebildeten Fixierschiebers kann vorteilhaft mit dem Kontrollelement, insbesondere mit einer oder mehrerer vierter Aussparungen, welche am Kontrollelement, vorzugsweise innerhalb der ersten Aussparung des Kontrollelements, angeordnet sind, wechselwirken und so eine Fixierung der Deviationsbewegung der Hand in einer gewünschten Winkelstellung ermöglichen.

Die vorliegende Erfindung betrifft darüber hinaus auch eine Orthese zur kontrollierten Bewegung und/oder Fixierung einer Hand oder einer Hand und eines Unterarms eines Patienten, mit einem einstellbaren Orthesengelenk wie zuvor beschrieben.

Diese sowie zusätzliche Einzelheiten und weitere Vorteile der Erfindung werden nachfolgend anhand bevorzugter Ausführungsbeispiele, auf welche die vorliegende Erfindung jedoch nicht beschränkt ist, und in Verbindung mit der beigefügten Zeichnung beschrieben.

Darin zeigen schematisch:
- Fig. 1: eine Ausgestaltung eines erfindungsgemäßen Orthesengelenks in einer perspektivischen Ansicht (Kontrollelement als Umrisszeichnung dargestellt);
- Fig. 2: Schnitt entlang einer Längsachse einer Orthese umfassend eine Ausgestaltung eines erfindungsgemäßen Orthesengelenks;
- Fig. 3: in den Teilfiguren a bis c eine Ausgestaltung eines erfindungsgemäßen ersten Stellmittels in einer Draufsicht (Fig. 3a), mit Blick auf eine Unterseite des ersten Stellmittels (Fig. 3b) und in einer Seitenansicht (Fig. 3c);
- Fig. 4: in Teilzeichnung eine weitere Ausgestaltung eines erfindungsgemäßen ersten Stellmittels in einer perspektivischen Ansicht auf eine Unterseite des ersten Stellmittels (Fig. 4a) bzw. in einer Ansicht mit Blick auf eine Unterseite des ersten Stellmittels (Fig. 4b), ein korrespondierend dazu ausgestalteter erster Kontrollschieber in einer Seitenansicht (Fig. 4c) sowie eine perspektivische Ansicht eines Kontrollelements mit einem derartig ausgestalteten ersten Stellmittel (Fig. 4d);
- Fig. 5a: in einer Draufsicht eine Ausgestaltung eines Kontrollelements mit einer Ausgestaltung eines ersten Kontrollschiebers und damit wirkverbundenem Schwenkhebel;

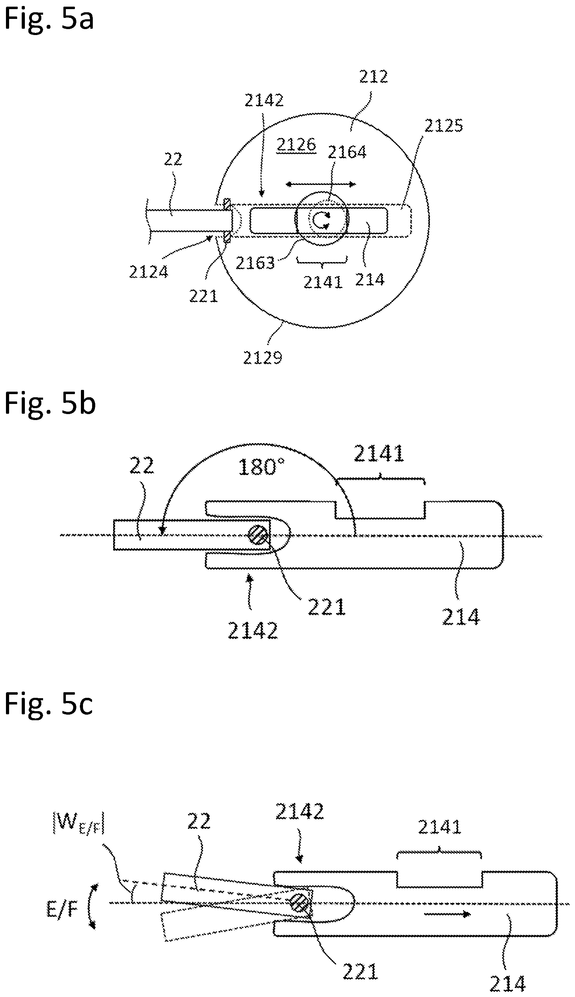

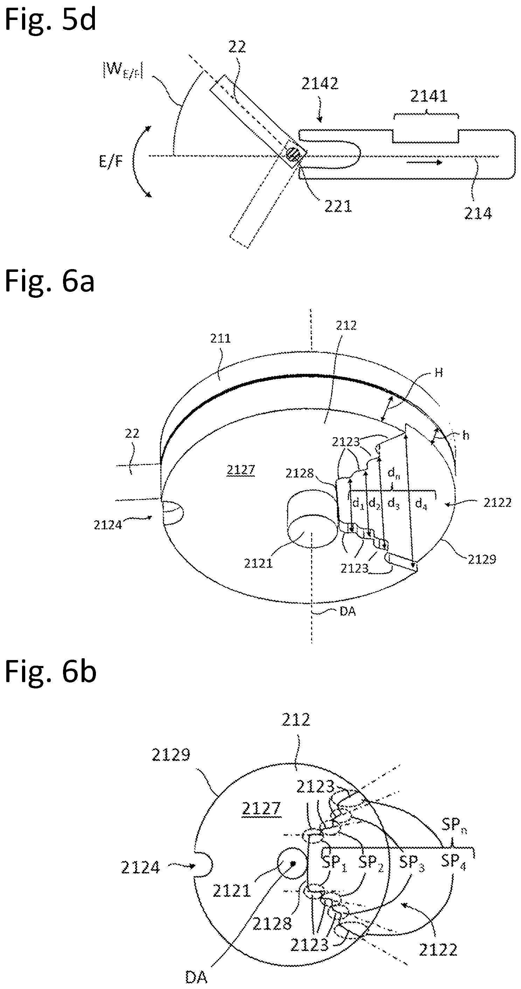

- Fig. 5 b - d: in den Teilfiguren 5b bis 5d jeweils in einer Seitenansicht drei mögliche Positionen des ersten Kontrollschiebers in Bezug zum Schwenkhebel zur Illustration der Wirkungsweise des ersten Kontrollschiebers bei der Kontrolle der Extension und Flexion der Hand;
- Fig. 6a: eine Ausgestaltung eines Kontrollelements sowie eines Deckelements in einer perspektivischen Ansicht der Unterseite des Kontrollelements;
- Fig. 6b: das Kontrollelement aus Fig. 6a mit Blick auf die Unterseite;

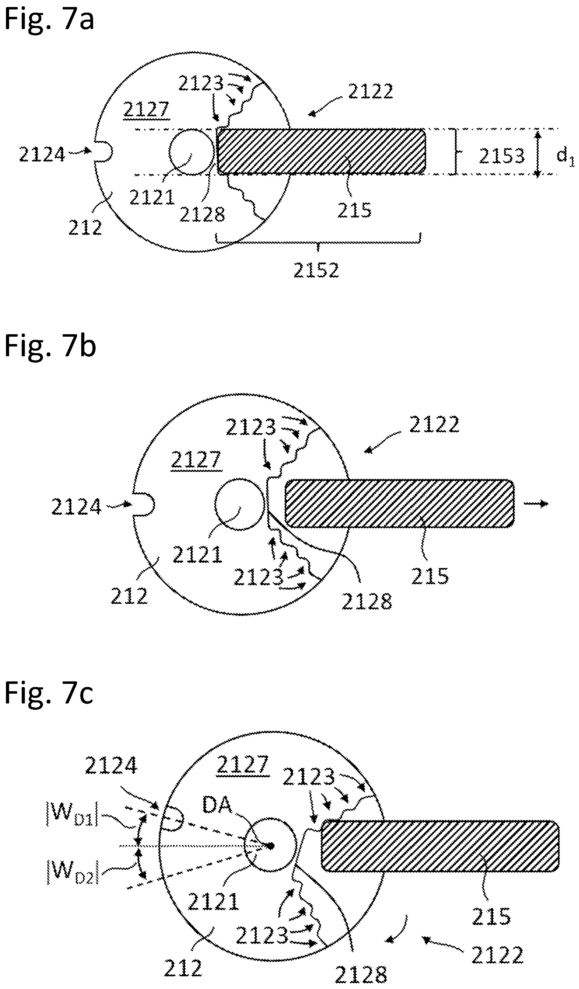

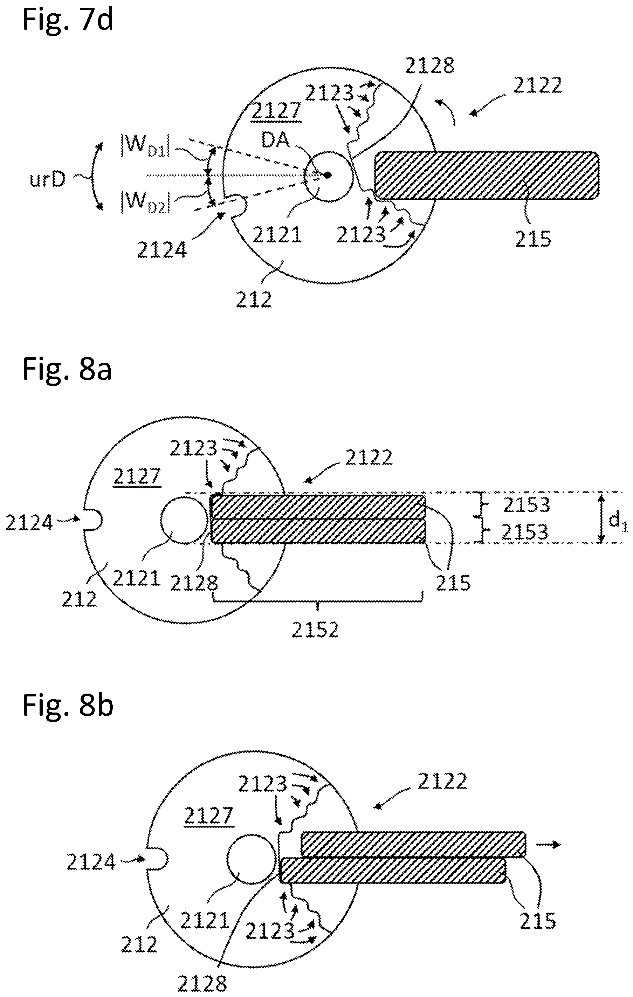

- Fig. 7: in den Teilfiguren a bis d das Zusammenspiel einer Ausgestaltung eines zweiten Kontrollschiebers mit dem Kontrollelement aus den Fig. 6 a und b zur Illustration der Kontrolle der Deviationsbewegung der Hand;

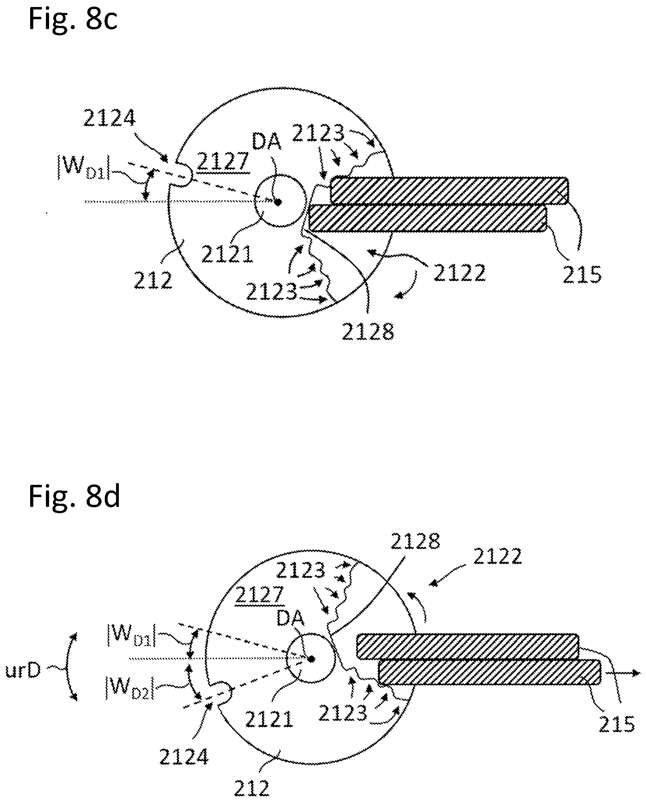

- Fig. 8: in den Teilfiguren a bis d das Zusammenspiel von zwei zweiten Kontrollschiebern mit dem Kontrollelement aus den Fig. 6 a und b zur Illustration der Kontrolle der Deviationsbewegung der Hand;

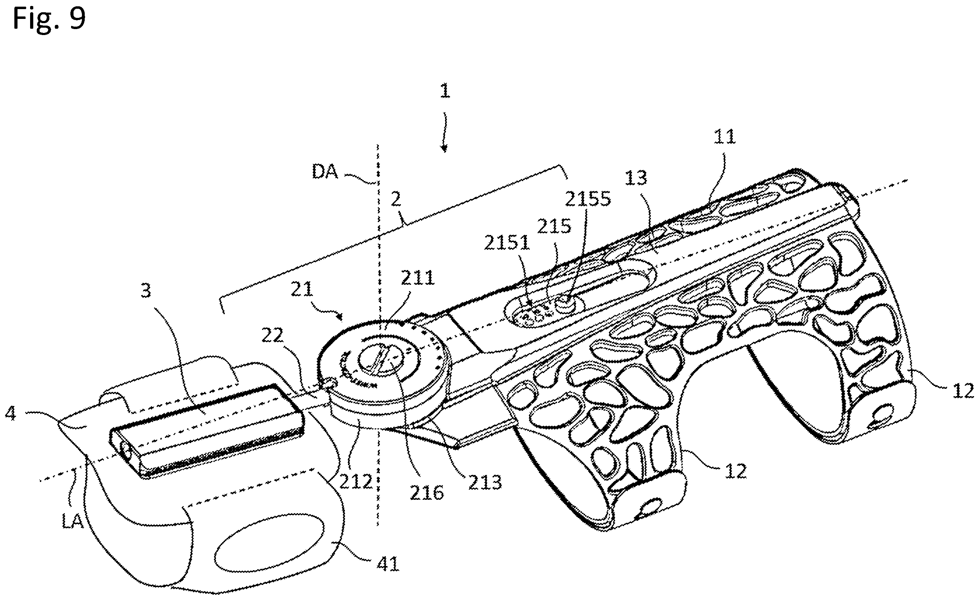

- Fig. 9: eine Ausgestaltung einer Orthese mit einer Ausgestaltung eines erfindungsgemäßen Orthesengelenks in einer perspektivischen Ansicht;

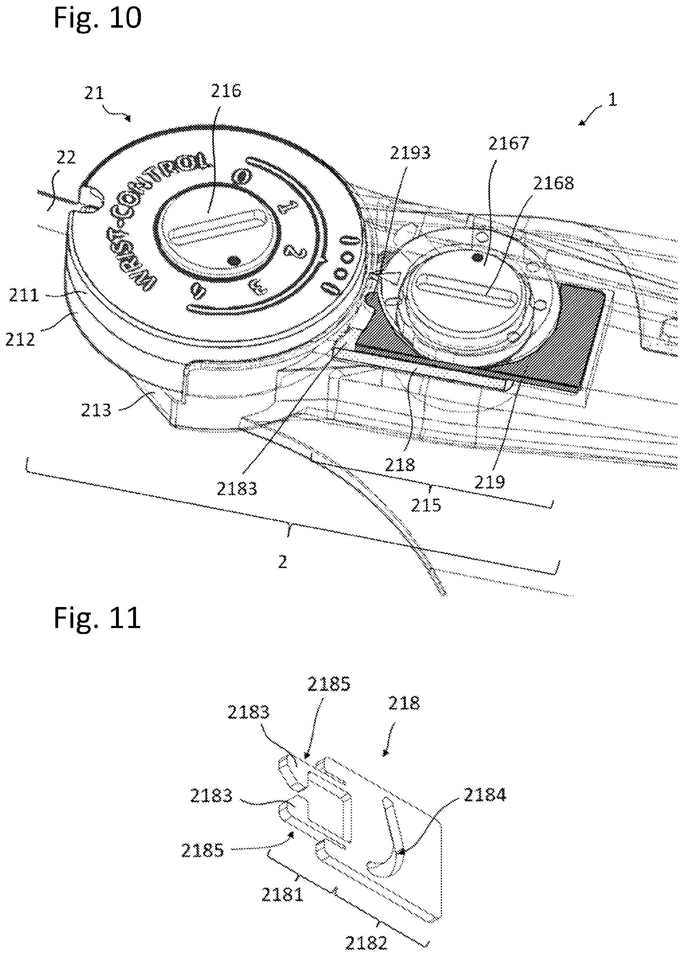

- Fig. 10: eine weitere Ausgestaltung eines erfindungsgemäßen Orthesengelenks mit zwei zweiten Kontrollschiebern, wobei einer der Kontrollschieber als ein Deviationskontrollschieber und der andere als ein Fixierschieber ausgebildet ist;
- Fig. 11: eine Ausgestaltung eines Deviationskontrollschiebers wie in Fig. 10 einzeln dargestellt;

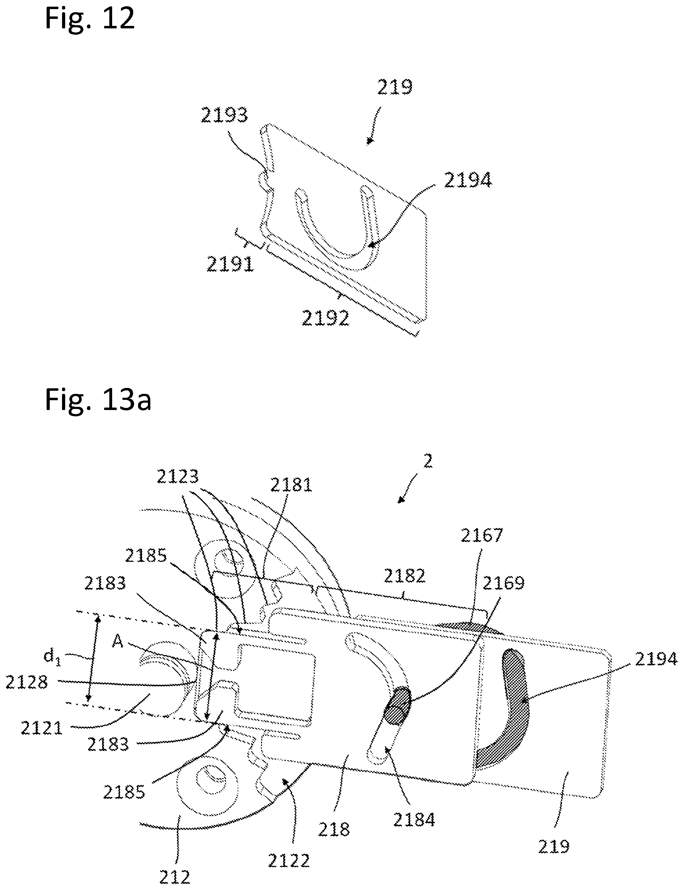

- Fig. 12: eine Ausgestaltung eines Fixierschiebers wie in Fig. 10 ebenfalls einzeln dargestellt;

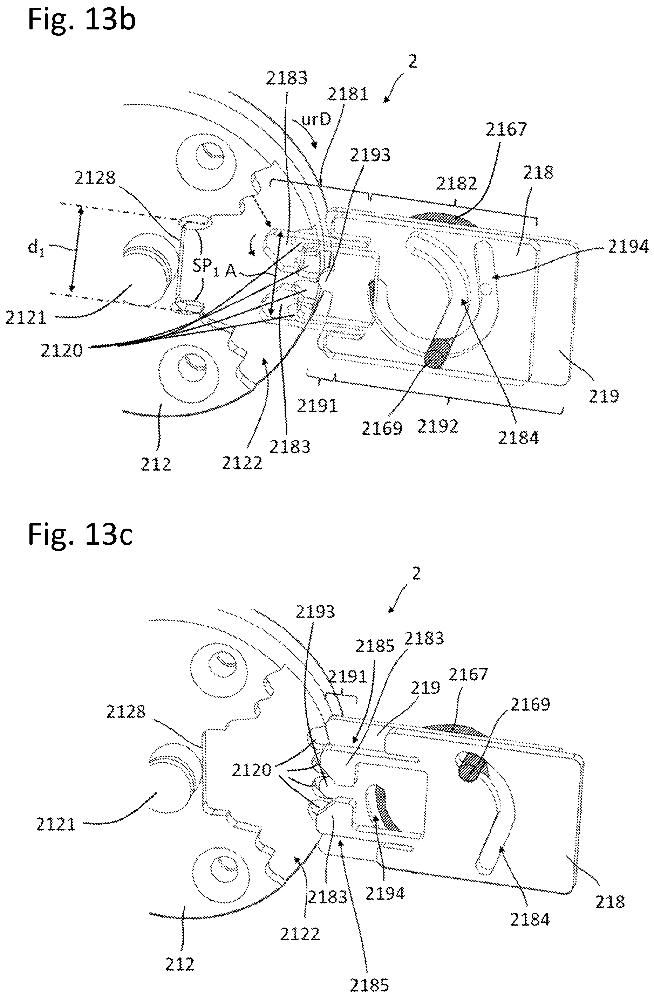

- Fig. 13: in den Teilfiguren a bis c das Zusammenspiel der in Fig. 10 gezeigten Ausgestaltung eines Orthesengelenks mit einem Deviationskontrollschieber und einem Fixierschieber zur Illustration der Kontrolle und Fixierung der Deviationsbewegung der Hand; und

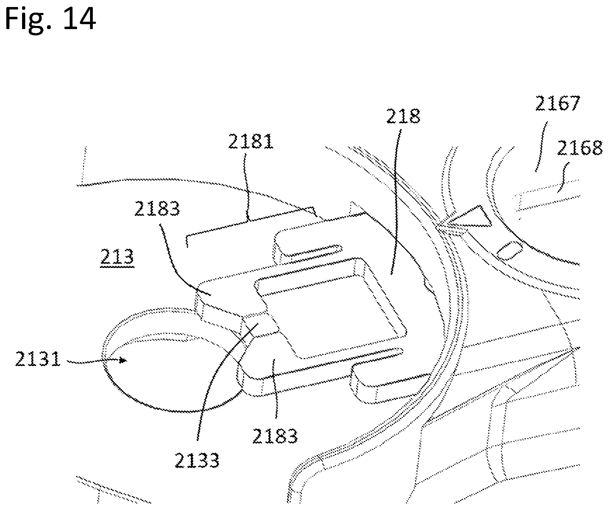

- Fig. 14: eine Ansicht einer Ausgestaltung eines Grundelements eines Orthesengelenks wie in Fig. 10 gezeigt sowie einen darin eingeschobenen Deviationskontrollschieber.

Bei der nachfolgenden Beschreibung bevorzugter Ausführungsformen der vorliegenden Erfindung bezeichnen gleiche Bezugszeichen gleiche oder vergleichbare Komponenten.

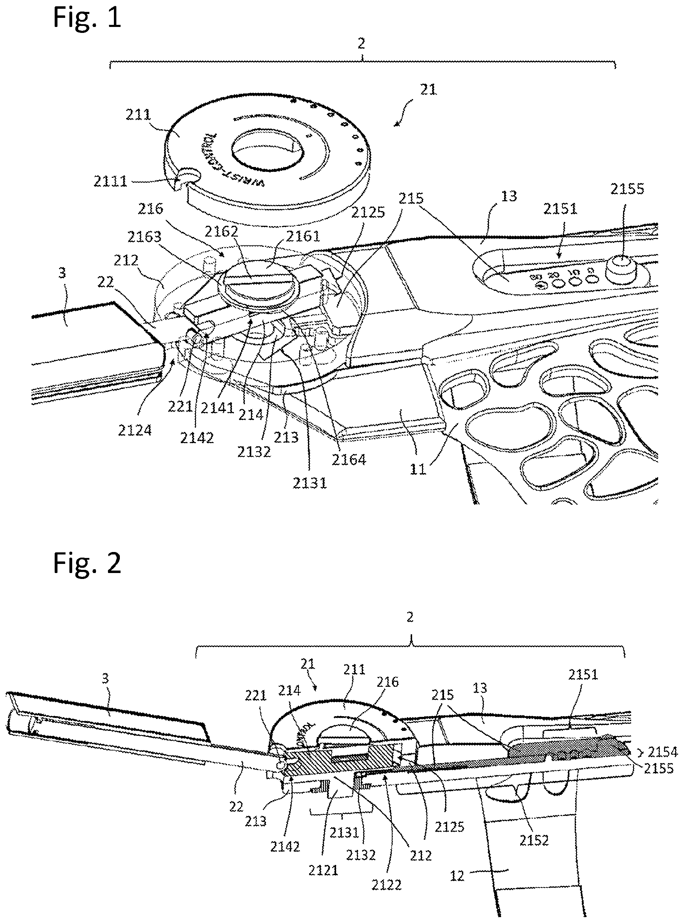

In Fig. 1 ist eine Ausgestaltung eines erfindungsgemäßen Orthesengelenks 2 in einer perspektivischen Ansicht dargestellt. Ein Kontrollelement 212 ist dabei als Umrisszeichnung dargestellt, um auch darunter liegende Bauteile sichtbar zu machen.

Ein erfindungsgemäßes, einstellbares Orthesengelenk 2 zur kontrollierten Bewegung und/oder Fixierung einer Hand eines Patienten umfasst wenigstens ein Bewegungsmodul 21 zur Kontrolle einer Extensions- und/oder Flexionsbewegung E/F sowie zur Kontrolle und Ausführung einer ulnaren und/oder radialen Deviation urD der Hand. Zur Ausführung der Extensions- und/oder Flexionsbewegung E/F der Hand ist ein Schwenkhebel 22 mit dem Bewegungsmodul 21 wirkverbunden. Der Schwenkhebel 22 kann dazu vorzugsweise über eine Schwenkachse 221 mit dem Kontrollelement 212 wirkverbunden bzw. daran schwenkbar gelagert sein. Das Bewegungsmodul 21 umfasst darüber hinaus wenigstens ein Grundelement 213 und wenigstens ein Kontrollelement 212 mit einer Oberseite 2126 und einer dem Grundelement 213 zugewandten und der Oberseite 2126 gegenüberliegenden Unterseite 2127. An der Unterseite 2127 ist ein Drehzylinder 2121 angeordnet, mittels dessen das Kontrollelement 212 gegenüber dem Grundelement 213 bezüglich einer Deviationsachse DA drehbar gelagert ist. Das Grundelement 213 kann dazu vorzugsweise eine Aufnahme 2131 für den Drehzylinder 2121 aufweisen. Das Kontrollelement 212 nebst Drehzylinder 2121 kann insbesondere aus einem metallischen Werkstoff, bevorzugt aus Aluminium, gefertigt sein. Zur drehbaren Lagerung des Kontrollelements 212 gegenüber dem Grundelement 213 kann zwischen Grundelement 213 und Drehzylinder 2121 darüber hinaus ein Führungsmittel 2132 für den Drehzylinder 2121 vorgesehen sein. Ein derartiges Führungsmittel 2132, welches vorzugsweise aus Stahl in Form einer Hülse gefertigt ist, kann vorteilhaft eine Reibung des metallischen Drehzylinders 2121 gegenüber dem Grundelement 213, insbesondere gegenüber einem aus einem Kunststoff hergestellten Grundelement 213, reduzieren (vgl. hierzu insb. Fig. 2).

Das Kontrollelement 212 ist erfindungsgemäß dazu eingerichtet, in seiner Drehbewegung um die Deviationsachse DA kontrolliert beschränkt zu werden und das Kontrollelement 212 ist zudem dazu eingerichtet; die Bewegung des Schwenkhebels 22 in seinem Bewegungsumfang kontrolliert zu beschränken. Die Schwenkachse 221, über die der Schwenkhebel 22 mit dem Kontrollelement 212 vorzugsweise wirkverbunden ist, kann dazu innerhalb einer Aussparung 2124 angeordnet sein, welche wiederum am Rand 2129 des Kontrollelements 212 angeordnet sein kann. Diese zweite Aussparung 2124 kann sich insbesondere über eine gesamte Höhe H des Kontrollelements 212 erstrecken, dem Schwenkhebel 22 so Bewegungsfreiheit für seine Schwenkbewegung senkrecht zur Ebene des Kontrollelements 212 ermöglichen und ist bevorzugt am gegenüber liegenden Rand 2129 zu einer an der Unterseite 2127 des Kontrollelements 212 angeordneten ersten Aussparung 2122 zur Deviationskontrolle angeordnet. Entsprechend kann auch in einem Deckelement 211 zur Abdeckung des Kontrollelements 212 eine Aussparung 2111 für den Schwenkhebel 22 vorgesehen sein.

Wie in **Fig. 1** zu sehen, umfasst das Kontrollelement 212 zudem wenigstens einen ersten Kontrollschieber 214, welcher mittels wenigstens eines ersten Stellmittels 216 bezüglich des mit dem Bewegungsmodul 21 wirkverbundenen Schwenkhebels 22 verschiebbar ist. Dazu kann das Kontrollelement 212 eine dritte Aussparung 2125 für den ersten Kontrollschieber 214 aufweisen, welche vorzugsweise an der Oberseite 2126 des Kontrollelements 212 angeordnet ist und insbesondere entlang einer Verbindungslinie von der Mitte der ersten Aussparung 2122 zur Deviationskontrolle zur Mitte der zweiten Aussparung 2124 für den Schwenkhebel 22 verläuft.

**Fig. 2** zeigt einen Schnitt entlang einer Längsachse LA einer Orthese 1 umfassend eine Ausgestaltung eines erfindungsgemäßen Orthesengelenks 2. In **Fig. 2** ist der erste Kontrollschieber 214 diagonal schraffiert hervorgehoben und es ist zu sehen, wie er innerhalb der dritten Aussparung 2125 geführt wird.

Der erste Kontrollschieber 214 kann, wie in den **Fig. 1** und **2** dargestellt, im Wesentlichen quaderförmig ausgestaltet und an seinem, dem Schwenkhebel 22 zugewandten Ende gabelförmig ausgebildet sein. Das gabelförmige Ende 2142 des ersten Kontrollschiebers 214 kann vorzugsweise so ausgestaltet sein, dass das mit der Schwenkachse 221 verbundene Ende des Schwenkhebels 22 zwischen die "Gabelzinken" geschoben werden kann, wobei der Umfang der Schwenkbewegung des Schwenkhebels 22 umso mehr eingeschränkt wird, je weiter das mit der Schwenkachse 221 verbundene Ende des Schwenkhebels 22 in den Raum zwischen den Zinken des gabelförmigen Endes 2142 aufgenommen ist. Die **Fig. 5b** bis **5d** illustrieren diesen Mechanismus exemplarisch. Das zur Bewegung des ersten Kontrollschiebers 214 innerhalb der dritten Aussparung 2125 vorgesehene erste Stellmittel 216 umfasst vorzugsweise wenigstens einen Kopf 2161 und einen Stellzylinder 2164, welche, vorzugsweise über einen Kragen 2163, miteinander verbunden sind, sodass sich ein Bauteil mit einer Längsachse 2165 bildet. Der Kragen 2163 kann insbesondere dazu dienen, das erste Stellmittel 216 innerhalb des Bewegungsmoduls 21 drehbar zu fixieren, wozu das Bewegungsmodul 21 ein Deckelement 211 umfassen kann, welches das Kontrollelement 212 nebst aller darauf angeordneter Bauteile nach oben, also auf der dem Grundelement 213 gegenüberliegenden Seite des Bewegungsmoduls 21, zumindest teilweise abdeckt. In **Fig. 2** ist beispielsweise zu sehen, dass das Deckelement 211 das Kontrollelement 212 sowie alle darauf angeordneten Bauteile bis auf den Kopf 2161 des ersten Stellmittels 216 nach oben abdeckt. Der Kragen 2163 befindet sich hier unterhalb des Deckelements 211 und verhindert ein Herausfallen des ersten Stellmittels 216 aus der in **Fig.1** gut erkennbaren Öffnung in der Mitte des Deckelements 211.

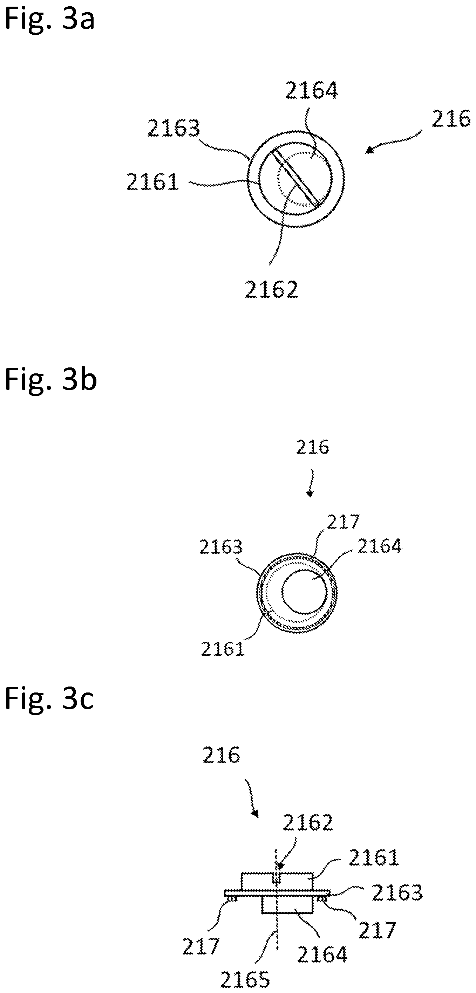

**Fig. 3** zeigt in den Teilfiguren **a** bis **c** eine Ausgestaltung eines erfindungsgemäßen ersten Stellmittels 216 in einer Draufsicht (**Fig. 3a**), mit Blick auf eine Unterseite des ersten Stellmittels 216 (**Fig. 3b**) und in einer Seitenansicht (**Fig. 3c**).

Der Stellzylinder 2164 des ersten Stellmittels 216 kann - wie hier zu sehen - einen kreisförmigen Querschnitt aufweisen und bezüglich der Längsachse 2165 außermittig mit dem Kopf 2161 des ersten Stellmittels 216 verbunden sein. Der Radius eines derartigen Stellzylinders 2164 kann dabei insbesondere kleiner sein als der Radius des Kopfes 2161. Der Stellzylinder 2164 kann zudem mit wenigstens einer, zur Aufnahme des Stellzylinders 2164 eingerichteten Aussparung 2141 des ersten Kontrollschiebers 214 wechselwirken. Dazu kann das erste Stellmittel 216 über eine Nut 2162, welche vorzugsweise an der Oberseite des Kopfes 2161 angeordnet ist, durch den Patienten selbst gedreht werden, wodurch der Stellzylinder 2164 mit einer Seitenbegrenzung der Aussparung 2141 in Wirkverbindung gelangt, Kraft auf besagte Seitenbegrenzung ausübt und dadurch den ersten Kontrollschieber 214 von der Rotationsachse des ersten Stellmittels 216 wegschiebt. Aufgrund der außermittigen Anordnung des Stellzylinders 2164 im Sinne einer Exzenterschraube verringert sich, wenn das erste Stellmittel 216 in die Gegenrichtung gedreht wird, die Wirkverbindung mit besagter Seitenbegrenzung und vergrößert sich mit einer gegenüberliegenden Seitenbegrenzung der Aussparung 2141, sodass der erste Kontrollschieber 214 entsprechend in die andere Richtung geschoben wird.

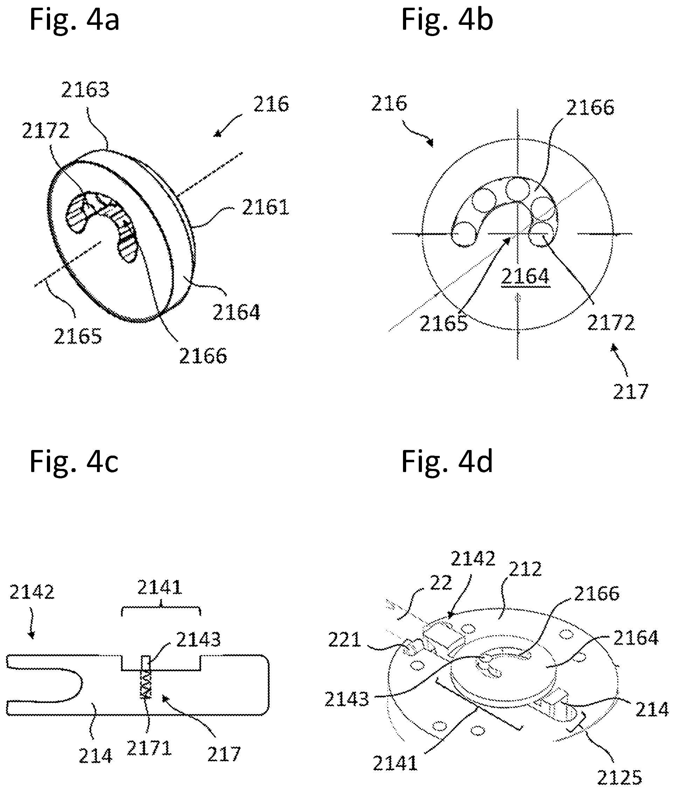

In **Fig. 4** ist in Teilzeichnung eine weitere Ausgestaltung eines erfindungsgemäßen ersten Stellmittels 216 in einer perspektivischen Ansicht auf eine Unterseite des ersten Stellmittels 216 (**Fig. 4a**) bzw. in einer Ansicht mit Blick auf eine Unterseite des ersten Stellmittels 216 (**Fig. 4b**) gezeigt, sowie ein korrespondierend dazu ausgestalteter erster Kontrollschieber 214 in einer Seitenansicht (**Fig. 4c**) und eine perspektivische Ansicht eines Kontrollelements 212 mit einem derartig ausgestalteten ersten Stellmittel 216 (**Fig. 4d**). In **Fig. 4d** ist das erste Stellmittel 216 dabei nicht komplett gezeigt, sondern es ist lediglich ein Schnitt durch den Stellzylinder 2164 eines ersten Stellmittels 216 wie in den **Fig. 4a** und **4b** abgebildet, um den Eingriff und die Führung des Verbindungstiftes 2143 innerhalb des Kanals 2166 zu illustrieren.

Alternativ zur zuvor beschriebenen Ausgestaltung kann das erste Stellmittel 216 auch wenigstens einen Kopf 2161 und einen Stellzylinder 2164 umfassen, welche, vorzugsweise über einen Kragen 2163, miteinander verbunden sind, sodass sich ein Bauteil mit einer Längsachse 2165 bildet, wobei der Stellzylinder 216 an seiner dem Kopf 2161 abgewandten Seite einen, vorzugsweise im Wesentlichen C-förmigen, Kanal 2166 aufweist. Der erste Kontrollschieber 214 kann in dieser Ausgestaltung insbesondere einen Verbindungsstift 2143 umfassen, welcher bei Gebrauch des Orthesengelenks 2 in den Kanal 2166 des Stellzylinders 2164 eingreift. Der Verbindungsstift 2143 kann dabei vorzugsweise innerhalb einer Aussparung 2141 des ersten Kontrollschiebers 214 angeordnet sein, welche eingerichtet ist, den Stellzylinder 2164 aufzunehmen (vgl. **Fig. 4c**). Der Kopf 2161 des ersten Stellmittels 216 kann wie zuvor mit einer Nut 2162 versehen sein, über die das erste Stellmittel 216 bezüglich seiner Längsachse 2165 gedreht werden kann. Bei einer Drehung eines derart ausgestalteten ersten Stellmittels 216 kann über den Verbindungsstift 2143, welcher in den im Wesentlichen C-förmigen Kanal 2166 des Stellzylinders 216 eingreift, durch Wechselwirkung mit den Wänden des Kanals 2166 vorteilhaft Kraft auf den ersten Kontrollschieber 214 ausgeübt werden (**Fig. 4d**). Je nach Drehrichtung kann der erste Kontrollschieber 214 dadurch in Richtung des Schwenkhebels 22 oder in Gegenrichtung innerhalb der dritten Aussparung 2125 des Kontrollelements 212 verschoben werden.

Zur Illustration der Wirkungsweise des ersten Kontrollschiebers 214 bei der Kontrolle der Extension und Flexion der Hand ist nun in **Fig. 5a** in einer Draufsicht eine Ausgestaltung eines Kontrollelements 212 mit einer Ausgestaltung eines ersten Kontrollschiebers 214 und damit wirkverbundenem Schwenkhebel 22 gezeigt und in den Teilfiguren **5b** bis **5d** jeweils in einer Seitenansicht drei mögliche Positionen des ersten Kontrollschiebers 214 in Bezug zum Schwenkhebel 22 während einer Benutzung.

Wie bereits beschrieben kann der erste Kontrollschieber 214, geführt durch die dritte, auf dem Kontrollelement 212 vorgesehene Aussparung 2125, bezüglich des Schwenkhebels 22 bzw. dessen Schwenkachse 221 mit Hilfe des ersten Stellmittels 216 verschoben werden. Exemplarisch ist dazu ein kreisförmiger, außermittig positionierter Stellzylinder 2164 gestrichelt in **Fig. 5a** angedeutet, ein Stellzylinder 2164 mit Kanal 2166, wie zuvor beschrieben (vgl. **Fig. 4a** und **4b**), wirkt allerdings entsprechend. **Fig. 5b** zeigt nun eine Position des ersten Kontrollschiebers 214, bei dem er vollständig zur Schwenkachse 221 des Schwenkhebels 22 hingeschoben ist und das gabelförmige Ende 2142 des ersten Kontrollschiebers 214 besagte Schwenkachse 221 sowie den daran anschließenden Teil des Schwenkhebels 22 vollständig einfasst. Die Gabelzinken des gabelförmigen Endes 2142 wirken als Anschlag und verhindern eine Schwenkbewegung des Schwenkhebels 22 um seine Schwenkachse 221. Der Winkel zwischen Schwenkhebel 22 und erstem Kontrollschieber 214 ist in dieser Position 180° (in **Fig. 5b** durch einen Winkelbogen und eine gestrichelte Linie angedeutet), was einer bezüglich des Unterarms ausgestreckten Hand eines Patienten entspricht und sozusagen eine Nullstellung darstellt. In dieser Position des ersten Kontrollschiebers 214 ist die Hand somit bezüglich einer Extensions- und Flexionsbewegung E/F fixiert. **Fig. 5c** zeigt eine Position des ersten Kontrollschiebers 214 in der er mittels des ersten Stellmittels 216 von der Schwenkachse 221 des Schwenkhebels 22 weggeschoben wurde. Diese Bewegung ist durch einen in **Fig. 5c** nach rechts weisenden Pfeil angedeutet. In dieser Position fasst das gabelförmige Ende 2142 des ersten Kontrollschiebers 214 den nahe der Schwenkachse 221 befindlichen Teil des Schwenkhebels 22 noch zu einem gewissen Grad, verhindert die Schwenkbewegung des Schwenkhebels 22 jedoch nicht mehr vollständig. Die Gabelzinken dienen wiederum als Anschlag und begrenzen die durch den Schwenkhebel 22 geführte Extensions- und Flexionsbewegung E/F der Hand bei einem vorgegebenen Winkel ± W_{E/F}. Der doppelte Betrag |W_{E/F}| dieses Winkels bestimmt dann den maximal mögliche Bewegungsumfang bei der Extensions- und Flexionsbewegung E/F der Hand des Patienten. **Fig. 5d** zeigt den ersten Kontrollschieber 214 schließlich in einer vollständig zum der Schwenkachse 221 gegenüberliegenden Ende der dritten Aussparung 2125 verschobenen Position. Das gabelförmige Ende 2142 erfasst den Schwenkhebel 22 und dessen Schwenkachse 221 nicht mehr. Die Schwenkbewegung des Schwenkhebels 22 und damit auch die Extensions- und Flexionsbewegung E/F der Hand erfolgt völlig ungehindert im anatomisch möglichen Bewegungsumfang. Durch die Wirkverbindung des Schwenkhebels 22 mit dem Kontrollelement 212 über die Schwenkachse 221 kann jedoch immer noch vorteilhaft eine Führung der Extensions- und Flexionsbewegung E/F der Hand erfolgen und so das Handgelenk des Patienten stabilisiert werden.

Das erste Stellmittel 216 und/oder der erste Kontrollschieber 214 können, insbesondere zur Gewährleistung einer sicheren und durch den Patienten selbst durchführbaren Einstellung des jeweils möglichen Bewegungsumfangs der Extensions- und Flexionsbewegung E/F der Hand, bevorzugt wenigstens einen Einrastmechanismus 217 umfassen, welcher eingerichtet ist, den ersten Kontrollschieber 214 in seiner Verschiebbewegung bezüglich des mit dem Bewegungsmodul 21 wirkverbundenen Schwenkhebels 22 an wenigstens einer vorgegebenen Position reversibel zu fixieren. Die **Fig. 4a** bis **d** zeigen eine Ausgestaltung eines derartigen Einrastmechanismus 217 am Beispiel eines ersten Stellmittels 216 mit Kanal 2166. Der an der dem Kopf 2161 abgewandten Seite des Stellzylinders 2164 angeordnete Kanal 2166 kann dazu Vertiefungen 2172 umfassen, welche an vorgegebenen Positionen innerhalb des Kanals 2166 angeordnet sind. Der am ersten Kontrollschieber 214 angeordnete Verbindungsstift 2143 kann dann bei Drehung des ersten Stellmittels 216 um dessen Längsachse 2165 in diesen Vertiefungen 2172 einrasten, bei Aufbringung eines geringen zusätzlichen Kraftaufwands jedoch auch wieder aus der jeweiligen Vertiefung 2172 herausbewegt und bis zur nächsten Vertiefung 2172 geführt werden. Das "Einrasten" in die einzelnen Vertiefungen 2172 kann der Patient beim Drehen des ersten Stellmittels 216 spüren. Es signalisiert ihm dadurch vorteilhaft eine korrekte Einstellung des Orthesengelenks 2. In einer bevorzugten Ausgestaltung eines derartigen Einrastmechanismus 217 kann der Verbindungsstift 2143 auch federnd am ersten Kontrollschieber 214 gelagert sein. In **Fig. 4c** ist hierzu exemplarisch eine Feder 2171 eingezeichnet. Besagte Feder 2171 kann vorteilhaft sowohl das Einrasten als auch das Lösen des Verbindungstiftes 2143 aus einer Vertiefung 2172 innerhalb des Kanals 2166 erleichtern. In einer Ausgestaltung des ersten Stellmittels 216 mit außermittig angeordnetem Stellzylinder 2164 wie in Fig. 3 gezeigt, kann der Einrastmechanismus 217 insbesondere als ein strukturierter, vorzugsweise gewellter Ring ausgestaltet sein, welcher an der dem Stellzylinder 2164 zugewandten Seite des Kragens 2163 angeordnet ist und mit einem korrespondierend dazu strukturierten Bereich des Kontrollmittels 212 und/oder des ersten Kontrollschiebers 214 wechselwirkt.

Die **Fig. 6a** und **6b** zeigen eine Ausgestaltung eines Kontrollelements 212 sowie eines Deckelements 211 in einer perspektivischen Ansicht der Unterseite 2127 des Kontrollelements 212 (**Fig. 6a**); bzw. einen Blick auf besagte Unterseite 2127 (**Fig. 6b**).

Das Kontrollelement 212 umfasst, wie bereits zuvor erwähnt, eine erste Aussparung 2122 zur Deviationskontrolle, welche an der Unterseite 2127 des Kontrollelements 212 angeordnet ist und sich vorzugsweise von einem Rand 2129 des Kontrollelements 212 in Richtung des Drehzylinders 2121 erstreckt. Eine Höhe h des Kontrollelements 212 kann dadurch im Bereich der ersten Aussparung 2122 kleiner als eine Höhe H des Kontrollelements 212 im Bereich außerhalb der ersten Aussparung 2122 sein, wodurch sich wenigstens vier Seitenkanten 2123 bilden, die den Bereich innerhalb der ersten Aussparung 2122 vom Bereich außerhalb der ersten Aussparung 2122 abgrenzen. Je zwei der Seitenkanten 2123 können in einem Abstand dₙ mit n = 1, 2, ... einander im Wesentlichen gegenüberliegen und ein Seitenkantenpaar SPₙ mit n = 1, 2, ... bilden. Ein Abstand dₙ zwischen zwei näher am Drehzylinder 2121 angeordneten, einander gegenüberliegenden Seitenkanten 2123 eines Seitenkantenpaares SPₙ ist dabei vorzugsweise stets kleiner als ein Abstand dₙ₊₁ zwischen zwei näher am Rand 2129 des Kontrollelements 212 angeordneten, einander gegenüberliegenden Seitenkanten 2123 eines Seitenkantenpaares SPₙ₊₁. Wie in den **Fig. 6a** und **b** zu sehen, kann die erste Aussparung 2122 auch durch eine Vielzahl von Seitenkanten 2123, vorzugsweise acht Seitenkanten 2123, gebildet werden, von denen je zwei einander im Wesentlichen gegenüberliegen und ein Seitenkantenpaar SPₙ mit n = 1, 2, ... bilden. Zum Drehzylinder 2121 hin kann die erste Aussparung 2122 insbesondere durch eine Endkante 2128 abgeschlossen sein. Die einander gegenüberliegenden Seitenkanten 2123 des ersten, benachbart dem Drehzylinder 2121 angeordneten Seitenkantenpaares SP₁ können, wie in **Fig. 6b** dargestellt, näherungsweise parallel zu einer Verbindungslinie verlaufen, welche die Mitte der zweiten Aussparung 2124 für den Schwenkhebel 22 und den Schnittpunkt der, durch den Drehzylinder 2121 verlaufenden Deviationsachse DA mit der Ebene des Kontrollelements 212 miteinander verbindet. Die Seitenkanten 2123 der Seitenkantenpaare SP₂, SP₃, SP₄, ... bilden bevorzugt jeweils einen Winkel mit dieser Verbindungslinie. In **Fig. 6b** sind die einzelnen Seitenkanten 2123 zur Verdeutlichung des jeweils entstehenden Winkels durch Strichpunkt-Linien verlängert.

Zur Kontrolle der ulnaren und radialen Deviationsbewegung urD der Hand wirkt nun ein zweiter Kontrollschieber 215 mit dem Kontrollelement 212 zusammen. Der zweite Kontrollschieber 215 kann dazu im Wesentlichen quaderförmig ausgebildet sein und eine Länge 2152, eine Breite 2153 und eine Höhe 2154 aufweisen. Die Breite 2153 des zweiten Kontrollschiebers 215 entspricht dabei bevorzugt dem Abstand d₁ der einander gegenüberliegenden Seitenkanten 2123 des ersten, am nächsten zum Drehzylinder 2121 angeordneten Seitenkantenpaares SP₁. Der zweite Kontrollschieber 215 kann darüber hinaus in die erste Aussparung 2122 des Kontrollelements 212 hineinführbar und herausführbar ausgestaltet sein und vorzugsweise mit den Seitenkanten 2123 wechselwirken.

**Fig. 7** zeigt in den Teilfiguren a bis d das Zusammenspiel einer Ausgestaltung eines zweiten Kontrollschiebers 215 mit dem Kontrollelement 212 aus den **Fig. 6** **a** und **b** zur Illustration der Kontrolle der Deviationsbewegung der Hand.

**Fig. 7a** zeigt schematisch die Unterseite 2127 des Kontrollelements 212 mit Drehzylinder 2121, erster Aussparung 2122 und zweiter Aussparung 2124 in einer Draufsicht. In die erste Aussparung 2122 ist der zweite Kontrollschieber 215 bis zur Endkante 2128 der ersten Aussparung 2122 hineingeführt. Die Breite 2153 des Kontrollschiebers 215 entspricht in dieser Ausgestaltung dem Abstand d₁ zwischen den einander gegenüberliegenden Seitenkanten 2123 des ersten Seitenkantenpaares SP₁. In dieser Position wechselwirken die Kanten des zweiten Kontrollschiebers 215 demnach mit den Seitenkanten 2123 des ersten Seitenkantenpaares SP₁, sodass eine Drehung des Kontrollelements 212 um die durch den Drehzylinder 2121 verlaufende Deviationsachse DA verhindert wird. Ein mit dem Kontrollelement 212 wirkverbundener Schwenkhebel 22 (hier nicht eingezeichnet, aber bspw. in den Fig. 1, 2, 5 und 9 zu sehen) kann sich somit ebenfalls nicht im Rahmen einer Drehbewegung bezüglich der Deviationsachse DA bewegen, sodass keine Deviationsbewegung der Hand zugelassen ist - die Hand ist bezüglich eine ulnare und radial Deviation urD fixiert.

In **Fig. 7b** ist der zweiten Kontrollschieber 215 in einer Position gezeigt, in der er ein wenig aus der ersten Aussparung 2122 herausgeführt und von der Endkante 2128 der ersten Aussparung 2122 weggeführt ist. In dieser Position besteht kein Kontakt mehr zu den Seitenkanten 2123 des ersten Seitenkantenpaares SP₁ und eine Drehbewegung des Kontrollelements 212 mittels des Drehzylinders 2121 um die Deviationsachse DA ist möglich (gebogene Pfeile in den **Fig. 7c** und **7d**). In den **Fig. 7c** und **7d** sind die Endpunkte besagter Drehbewegungen dargestellt, wobei **Fig. 7c** den Endpunkt einer radialen Deviationsbewegung (Radialabduktion) und **Fig. 7d** den Endpunkt einer ulnaren Deviationsbewegung (Ulnarabduktion) zeigt. Die Drehbewegung um die Deviationsachse DA in eine Richtung wird dabei durch das erneute Auftreten einer Wechselwirkung zwischen der entsprechenden Kante des zweiten Kontrollschiebers 215 und der jeweiligen Seitenkante 2123, hier exemplarisch des zweiten Seitenkantenpaares SP₂, beendet. Die Beträge der überstrichenen Winkel |W_{D1}| (Radialabduktion) und |W_{D2}| (Ulnarabduktion) sind in dieser Ausgestaltung gleich. Als Bewegungsumfang für die gesamte, in dieser Position des zweiten Kontrollschiebers 215 zugelassene Deviationsbewegung urD ergibt sich die Summe aus |W_{D1}| und |W_{D2}|, hier also 2 x |W_{D1}|. Durch weiteres Herausführen des zweiten Kontrollschiebers 215 aus der ersten Aussparung 2122 kann der mögliche, vom Kontrollelement 212 zugelassene Bewegungsumfang vergrößert werden. Aus den **Fig. 7c** und **7d** ist ersichtlich, dass wenn der zweite Kontrollschieber 215 etwas weiter vom Drehzylinder 2121 bzw. der Endkante 2128 entfernt wird, bei einer Drehung des Kontrollelements 212 um die Deviationsachse DA nicht mehr die Seitenkanten 2123 des zweiten Seitenkantenpaares SP₂ mit den jeweiligen Kanten des zweiten Kontrollschiebers 215 wechselwirken würden, sondern die Seitenkanten 2123 der weiter zum Rand 2129 des Kontrollelements 212 angeordneten Seitenkantenpaare SP₃ oder SP₄. Der Betrag des insgesamt überstrichenen Winkels |W_{D1}| + |W_{D2}| wäre entsprechend größer.

Die Bezeichnungen der einzelnen Seitenkantenpaare SPₙ wurden der Übersichtlichkeit wegen in den **Fig 7a** - **d** und **8 a** - **d** weggelassen, sie können jedoch **Fig. 6b** entnommen werden.

Alternativ zu der in den **Fig. 7a** - **7d** dargestellten Ausgestaltung des erfindungsgemäßen Orthesengelenks 2 können statt einem auch zwei, mit dem Kontrollelement 212 zusammenwirkende zweite Kontrollschieber 215 für die Kontrolle der ulnaren bzw. radialen Deviationsbewegung urD der Hand vorgesehen sein, wobei die zweiten Kontrollschieber 215 jeweils im Wesentlichen quaderförmig ausgebildet sind und eine Länge 2152, eine Breite 2153 und eine Höhe 2154 aufweisen können. In dieser Ausgestaltung entspricht bevorzugt die Summe der Breiten 2153 dem Abstand d₁ der einander gegenüberliegenden Seitenkanten 2123 des ersten, am nächsten zum Drehzylinder 2121 angeordneten Seitenkantenpaares SP₁. Ein jeder zweite Kontrollschieber 215 kann zudem in die erste Aussparung 2122 des Kontrollelements 212 hineinführbar und herausführbar ausgestaltet sein und vorzugsweise mit den Seitenkanten 2123 wechselwirken.

**Fig. 8** zeigt in den Teilfiguren **a** bis **d** wiederum das Zusammenspiel von zwei zweiten Kontrollschiebern 215 mit dem Kontrollelement 212 aus den **Fig. 6** **a** und **b** zur Illustration der Kontrolle der Deviationsbewegung der Hand.

**Fig. 8a** entspricht der in **Fig. 7a** dargestellten Situation: beide zweiten Kontrollschieber 215 sind bis zur Endkante 2128 der ersten Aussparung 2122 in die erste Aussparung 2122 hineingeführt. Da die Summe der Breiten 2153 der zweiten Kontrollschieber 215 dem Abstand d₁ der einander gegenüberliegenden Seitenkanten 2123 des ersten, am nächsten zum Drehzylinder 2121 angeordneten Seitenkantenpaares SP₁ entspricht, wechselwirken beide Kontrollschieber 215 mit der ihnen jeweils nächsten Seitenkante 2123 des ersten Seitenkantenpaares SP₁ und eine Drehung des Kontrollelements 212 um die durch den Drehzylinder 2121 verlaufende Deviationsachse DA wird verhindert. Die hier gezeigte Ausgestaltung mit zwei zweiten Kontrollschiebern 215 ermöglicht nun zum einen den geleichen Kontrollmechanismus für eine Kontrolle der ulnaren und radialen Deviationsbewegung urD der Hand, wie die in den **Fig. 7a** - **d** dargestellte Ausgestaltung. Voraussetzung dafür ist, dass die beiden zweiten Kontrollschieber 215 stets gleiche Positionen innerhalb der ersten Aussparung 2122 einnehmen, also stets gleichweit in die erste Aussparung 2122 hineingeführt- bzw. herausgeführt sind. Für diesen Kontrollmechanismus wird auf die Beschreibung zu den **Fig. 7a - 7d** verwiesen.

Zum anderen ermöglicht die hier gezeigte Ausgestaltung mit zwei zweiten Kontrollschiebern 215 jedoch auch eine unsymmetrische Kontrolle der Deviationsbewegung urD der Hand. In den **Fig. 8b** und **8c** ist dazu exemplarisch eine Situation dargestellt, in der einer der zweiten Kontrollschieber 215 ein wenig in Richtung des Randes 2129 des Kontrollelements 212 aus der ersten Aussparung 2122 herausgeführt ist, der andere zweite Kontrollschieber 215 in seiner ursprünglichen Position mit Kontakt zur Endkante 2128 der ersten Aussparung 2122 verbleibt. Bei einer derartigen Positionierung der zwei zweiten Kontrollschieber 215 kann das Kontrollelement 212 mittels des Drehzylinders 2121 lediglich eine Drehung bezüglich der Deviationsachse DA im Rahmen einer radialen Deviation (entsprechend einer Radialabduktion) ausführen, eine Drehbewegung über die Ausgangsposition (**Fig. 8b**) hinaus in Richtung einer ulnaren Deviation (entsprechend einer Ulnarabduktion) wird durch die Wechselwirkung zwischen einer Kante des in seiner ursprünglichen Position verbliebenen zweiten Kontrollschiebers 215 mit der ihm naheliegenden Seitenkante 2123 des ersten Seitenkantenpaares SP₁ verhindert. Der Betrag des im Rahmen der radialen Deviationsbewegung maximal möglichen überstrichenen Winkels |W_{D1}| wird, wie in **Fig. 7c** für die Ausgestaltung mit einem zweiten Kontrollschieber 215 gezeigt und beschrieben, durch die Wechselwirkung einer Kante des zuvor verschobenen zweiten Kontrollschiebers 215 mit der Seitenkante 2123 des zweiten Seitenkantenpaares SP₂ bestimmt. Soll der Betrag des überstreichbaren Winkels |W_{D1}| größer sein, kann der zweite Kontrollschieber 215 weiter von der Endkante 2128 der ersten Aussparung 2122 wegbewegt werden und dann entsprechend mit der Seitenkante 2123 des dritten SP₃ oder vierten SP₄ Seitenkantenpaares wechselwirken. Entsprechend kann natürlich auch der in den **Fig. 8a** - **8d** oberer zweite Kontrollschieber 215 in seiner Ausgangsposition (vgl. **Fig. 8a**) verbleiben und der in den **Fig. 8a** - **8d** untere zweite Kontrollschieber 215 aus der ersten Aussparung 2122 herausgeführt werden, um so die radiale Deviation zu blockieren, die ulnare Deviation (entsprechend einer Ulnarabduktion) jedoch zuzulassen. In **Fig. 8d** ist schließlich eine Situation gezeigt, in der beide zweiten Kontrollschieber 215 aus ihrer Ausgangsposition in **Fig. 8a** in Richtung des Randes 2129 des Kontrollelements 212 aus der ersten Aussparung 2122 herausgeführt wurden, wobei die Verschiebung innerhalb der ersten Aussparung 2122 der beiden zweiten Kontrollschieber 215 unterschiedlich weit erfolgt ist. Der in **Fig. 8d** obere zweiten Kontrollschieber 215 befindet sich in einer Position innerhalb der ersten Aussparung 2122, die eine Wechselwirkung mit den Seitenkanten 2123 des zweiten Seitenkantenpaares SP₂ ermöglicht, wohingegen der in **Fig. 8d** untere zweiten Kontrollschieber 215 etwas weiter zum Rand 2129 verschoben wurde, sodass eine Wechselwirkung mit den Seitenkanten 2123 des dritten Seitenkantenpaares SP₃ ermöglicht wird. Eine Drehung des Kontrollelements 212 mittels des Drehzylinders 2121 um die Deviationsachse DA überstreicht in dieser Positionierung der zwei zweiten Kontrollschieber 215 einen unterschiedlichen Winkelbereich für die radiale Deviation (Winkelbetrag |W_{D1}|) und für die ulnare Deviation (Winkelbetrag |W_{D2}|). In **Fig. 8d** sind diese beiden unterschiedlich großen Winkel mittels gestrichelter Linien und gebogenen Doppelpfeilen gekennzeichnet. Der insgesamt mögliche Bewegungsumfang der ulnaren und radialen Deviation urD ergibt sich bei der hier dargestellten Positionierung der beiden zweiten Kontrollschieber 215 wieder aus der Summe der Beträge |W_{D1}| und |W_{D2}|, wobei im vorliegenden Beispiel der zugelassene Bewegungsumfang der ulnaren Deviation (entsprechend |W_{D2}|) größer ist als der zugelassene Bewegungsumfang der radialen Deviation (entsprechend |W_{D1}|). Durch verschiedene Positionierung der beiden zweiten Kontrollschieber 215 innerhalb der ersten Aussparung 2122 können somit vorteilhaft eine Vielzahl verschiedener Bewegungsumfänge der ulnaren und radialen Deviation urD kontrolliert werden, was eine individuelle Anpassung an den jeweiligen Gesundheits- bzw. Rehabilitationszustand des Patienten ermöglicht.

In **Fig. 9** ist eine Ausgestaltung einer erfindungsgemäßen Orthese 1 mit einer Ausgestaltung eines erfindungsgemäßen Orthesengelenks 2 in einer perspektivischen Ansicht gezeigt.

In dieser Ausgestaltung der Orthese 1 ist das Orthesengelenk 2 insbesondere über das Grundelement 213 des Bewegungsmoduls 21 mit einer Unterarmbefestigung 11 der Orthese 1 verbunden. Alternativ dazu kann auch ein Teil der Unterarmbefestigung 11 das Grundelement 213 eines Bewegungsmoduls 21 bilden und insbesondere eine Aufnahme 2131 für den Drehzylinder 2121 des Kontrollelements 212 aufweisen (nicht gezeigt). Die Orthese 1 bzw. deren Unterarmbefestigung 11 kann zudem über Befestigungsmittel 12 am Unterarm des Patienten reversibel befestigt werden. Als Befestigungsmittel 12 eignen sich insbesondere Klettbänder oder Gurte mit Perforierung und Gurtschnalle im weitesten Sinn. Die Befestigungsmittel 12 können jedoch auch durch ein Verfahren der additiven Fertigung ("3D-Druck") beispielsweise als bandartige Fortsetzung der Unterarmbefestigung 11 hergestellt und mit einem Schließmechanismus (bspw. durch Anordnung von Noppen auf einer Seite der bandartigen Fortsetzung und korrespondierend dazu ausgebildeten Löchern auf einer anderen bandartigen Fortsetzung der Unterarmbefestigung 11) versehen sein, wie es in **Fig. 9** exemplarisch dargestellt ist. Die Unterarmbefestigung 11 und das oder die Befestigungsmittel 12 können dabei vorzugsweise aus einem Kunststoff gefertigt sein.

Zur Gewährleistung einer sicheren, schnellen und ohne Verwendung von Werkzeug durch den Patienten selbst durchführbaren Einstellung vorgegebener Deviationswinkel für eine maximal mögliche Ulnar- und/oder Radialabduktion (bzw. ulnare/radiale Deviationsbewegung urD) kann ein jeder zweite Kontrollschieber 215 darüber hinaus eingerichtet sein, an vorgegebenen Positionen während des Hineinführens in und Herausführens aus der ersten Aussparung 2122 reversibel fixiert zu werden. Dazu kann vorzugsweise ein Einrastmechanismus 2151 vorgesehen sein. Ein jeder zweite Kontrollschieber 215 kann insbesondere an seiner zur Orthese 1 hin gerichteten Seite ein Sägezahnprofil aufweisen (nicht gezeigt), welches mit einer korrespondierend ausgestalteten, zum zweiten Kontrollschieber 215 hin gerichteten Seite der Orthese 1 und/oder eines Gehäuses 13 für den oder die zweiten Kontrollschieber 215 reversibel in Wirkverbindung treten und so einen Einrastmechanismus 2151 bilden kann. Eine Ausgestaltung eines Gehäuses 13 für den zweiten Kontrollschieber 215 ist exemplarisch in den **Fig. 1, 2** und **9** dargestellt. Der zweite Kontrollschieber 215 kann zur einfachen Bedienung durch den Patienten zudem ein Bedienelement 2155 aufweisen, mittels dessen er insbesondere innerhalb des Gehäuses 13 zum Bewegungsmodul 21 und damit zur ersten Aussparung 2122 des Kontrollelements 212 hin bzw. von dem Bewegungsmodul 21 und damit von der ersten Aussparung 2122 weggeführt werden kann.

Alternativ oder kumulativ kann der Einrastmechanismus 2151 auch durch einen oder mehrere, vorzugsweise gefederte, Stifte oder Noppen gebildet werden, welche an der zum zweiten Kontrollschieber 215 hin gerichteten Seite der Orthese 1 und/oder des Gehäuses 13 für den zweiten Kontrollschieber 215 angeordnet sein können und mit korrespondierend ausgebildeten Vertiefungen, insbesondere Löchern, in der zur Orthese 1 hin gerichteten Seite des oder der zweiten Kontrollschieber 215 wechselwirken. In den **Fig. 1** und **2** ist ein derartiger Einrastmechanismus 2151 exemplarisch gezeigt.

Zur Benutzung einer erfindungsgemäßen Orthese 1 durch einen Patienten kann der mit dem Kontrollelement 212 des Bewegungsmoduls 21 wirkverbundene Schwenkhebel 22, wie in **Fig. 9** dargestellt, besonders bevorzugt über ein Verbindungsmittel 3 mit einer Handbefestigung 4 verbunden sein. Das Verbindungsmittel 3 kann dabei mit der Handbefestigung 4 fest verbunden oder aber, beispielsweise über ein Klick- oder Einrastmechanismus, reversibel verbindbar ausgestaltet sein. Gegenüber dem Schwenkhebel 22 kann das Verbindungsmittel 3 vorzugsweise verschiebbar gelagert sein, wodurch vorteilhaft einer Veränderung des Abstands zwischen dem Bewegungsmodul 21 und einem Kontaktpunkt zwischen Handbefestigung 4 und Handoberseite bzw. Hand während der Ausführung einer Extensions- und Flexionsbewegung E/F Rechnung getragen werden kann.

Zur reversiblen Verbindung der Hand eines Patienten mit der Handbefestigung 4, kann an der Handbefestigung 4 schließlich ein Befestigungsmittel 41 anordenbar sein, wobei das Befestigungsmittel 41 insbesondere aus einem textilen Material gebildet sein kann, welches vorzugsweise über eine Klettverbindung mit der Unterseite der Handbefestigung 4 verbindbar ausgestaltet ist. Das Befestigungsmittel 41 kann dabei vorteilhaft den Daumen des Patienten umschließen, beispielsweise durch ein Loch innerhalb des textilen Materials des Befestigungsmittels 41 durch das der Daumen des Patienten gesteckt und dadurch umschlossen werden kann (**vgl.** **Fig. 9**) oder aber durch ein Befestigungsmittel 41, welches aus mehreren textilen Bändern gebildet wird, welche entsprechend um den Daumen des Patienten gelegt werden können (nicht gezeigt). Ein derart ausgestaltetes Befestigungsmittel 41 verhindert vorteilhaft das Rutschen der Orthese 1 bei Bewegungen des Patienten.

In **Fig. 10** ist eine weitere, alternative Ausgestaltung eines erfindungsgemäßen Orthesengelenks 2 mit zwei zweiten Kontrollschiebern 215 gezeigt, wobei einer der Kontrollschieber 215 als ein Deviationskontrollschieber 218 und der andere als ein Fixierschieber 219 ausgebildet ist.

In dieser Ausgestaltung wirken also für die Kontrolle der ulnaren/radialen Deviationsbewegung urD der Hand mit dem Kontrollelement 212 vorzugsweise zwei zweite Kontrollschieber 215 - ein als Deviationskontrollschieber 218 ausgebildeter und ein als Fixierschieber 219 ausgebildeter - zusammen.

In den **Fig. 11** und **12** sind Ausgestaltungen eines Deviationskontrollschiebers 218 und eines Fixierschiebers 219 jeweils einzeln dargestellt.

Der als Deviationskontrollschieber 218 ausgebildete zweite Kontrollschieber 215 kann, wie gezeigt, vorzugsweise einen Kopfbereich 2181 und einen Körperbereich 2182 umfassen, wobei der Kopfbereich 2181 eingerichtet ist, in die erste Aussparung 2122 des Kontrollelements 212 hineingeführt und herausgeführt zu werden und mit den Seitenkanten 2123 der ersten Aussparung 2122 des Kontrollelements 212 wechselzuwirken. Der Körperbereich 2182 des Deviationskontrollschiebers 218 umfasst vorzugsweise eine Führungsbahn 2184 für einen Kontrollstift 2169 eines, vom Orthesengelenk 2 umfassten, zweiten Stellmittels 2167. Das zweite Stellmittel 2167 kann beispielsweise als eine Art Schraube ausgestaltet sein, welche sich über eine Nut 2168 im Uhrzeigersinn und gegen den Uhrzeigersinn drehen lässt. Die Führungsbahn 2184 kann bevorzugt näherungsweise L-förmig ausgebildet sein, wobei der Übergang vom längeren Teil des "L" zum kürzeren Teil des "L" vorzugsweise rund ausgebildet sein und wobei der kürzere Teil des "L" insbesondere zum Kopfbereich 2181 des Deviationskontrollschiebers 218 hin ausgerichtet sein kann.

Der Kopfbereich 2181 des Deviationskontrollschiebers 218 kann besonders bevorzugt von zwei, einander gegenüberliegenden Hebelelementen 2183 gebildet werden, welche gegeneinander federnd bewegbar sind. Die Federwirkung der Hebelelemente 2183 kann dabei insbesondere einerseits durch die bis zu einem gewissen Grad vorhandenen elastischen Eigenschaften des verwendeten Materials, aus dem der Deviationshebel 218 gefertigt ist, erzeugt werden. Hierfür kann beispielsweise ein metallischer Werkstoff, insbesondere Aluminium, verwendet werden. Andererseits können die Hebelelemente 2183 - alternativ und kumulativ - auch baulich so ausgestaltet sein, dass eine gewisse Elastizität erzeugt wird. In Fig. 11 ist exemplarisch gezeigt, dass die Hebelelemente 2183 insbesondere als Zinken einer Gabel ausgestaltet sein können, welche vom Körperbereich 2182 des Deviationskontrollschiebers 218 wegzeigen. Die jeweilige schmale Ausgestaltung der Hebelelemente 2183 insbesondere im Übergang zum Körperbereich 2182 erzeugt hier vorteilhaft eine gewisse Federwirkung der jeweiligen Hebelelemente 2183 bei Einwirkung von außen. In einem entspannten Zustand, also ohne äußere Einwirkung auf die Hebelelemente 2183, können die Außenkanten 2185 besagter Hebelelemente 2183 zueinander zudem bevorzugt einen Abstand A aufweisen, der dem Abstand d₁ der einander gegenüberliegenden Seitenkanten 2123 des ersten, am nächsten zum Drehzylinder 2121 angeordneten Seitenkantenpaares SP₁ der ersten Aussparung 2122 entspricht.

Der andere, als Fixierschieber 219 ausgebildete zweite Kontrollschieber 215 umfasst vorzugsweise ebenso wenigstens einen Kopfbereich 2191 und einen Körperbereich 2192, wobei der Kopfbereich 2191 des Fixierschiebers 219 insbesondere dazu eingerichtet ist mit einer vierten Aussparung 2120 des Kontrollelement 212 wechselzuwirken. Das erfindungsgemäße Orthesengelenk 2 umfasst in dieser Ausgestaltung vorzugsweise wenigstens eine, am Kontrollelement 212 angeordnete, vierte Aussparung 2120 zur Deviationsfixierung. Bevorzugt sind allerdings eine Vielzahl von vierten Aussparungen 2120 vorgesehen, wie exemplarisch in den Fig. 13 b und 13 c durch vier derartige vierte Aussparungen 2120 angedeutet ist.

Der Kopfbereich 2191 des Fixierschiebers 219 kann zur Wechselwirkung mit besagter vierter Aussparung 2120, wie in Fig. 12 dargestellt, ein Kopfelement 2193 umfassen, welches fest mit dem Körperbereich 2192 des Fixierschiebers 219 verbunden ist. Abseits des Kopfelements 2193 kann der Kopfbereich 2191 des Fixierschiebers 219 zudem konkav ausgebildet sein, sodass bei einer Wechselwirkung des Kopfelements 2193 mit der vierten Aussparung 2120 auch der übrige Kopfbereich 2191 des Fixierschiebers 219 mit dem Kontrollelement 212 in Kontakt kommen kann (vgl. dazu Fig. 13 c). Ein derartiger Kontakt ermöglicht vorteilhaft eine weitere, gleichmäßiger verteilte Kraftübertragung vom Kontrollelement 212 auf den Fixierschieber 219 und verstärkt somit vorteilhaft die Fixierung der Deviationsbewegung urD, wenn das Kopfelement 2193 in die vierte Aussparung 2120 eingreift.

Der Körperbereich 2192 des Fixierschiebers 219 kann wiederum eine Führungsbahn 2194 für den Kontrollstift 2169 des zweiten Stellmittels 2167 aufweisen. Besagte Führungsbahn 2194 kann, wie in Fig. 12 zu sehen, vorzugsweise U-förmig ausgebildet sein, wobei die Öffnung des "U" vorzugsweise in Richtung hin zu einer Längsseite des Fixierschiebers 219 gerichtet ist und wobei der dem Kopfbereich 2191 näher liegende Arm des "U" bevorzugt kürzer als der vom Kopfbereich 2191 weiter entfernt liegende Arm des "U" sein kann.

**Fig. 13** zeigt nun in den Teilfiguren a bis c das Zusammenspiel der in Fig. 10 gezeigten Ausgestaltung eines Orthesengelenks 2 mit einem Deviationskontrollschieber 218 und einem Fixierschieber 219 zur Illustration der Kontrolle und Fixierung der Deviationsbewegung der Hand.

**Fig. 13** **a** zeigt exemplarisch die Unterseite 2127 des Kontrollelements 212 mit einem bis zur Endkante 2128 der ersten Aussparung 2122 eingeschobenen Deviationskontrollschieber 218. Bleibt das Kontrollelement 212 bezüglich des Deviationsschiebers 218 in Ruhe, wie hier gezeigt, entspricht der Abstand A zwischen den beiden Hebelelementen 2183 vorzugsweise dem Abstand d₁ zwischen den beiden Seitenkanten 2123, die am nächsten zum Drehzylinder 2121 liegen, also dem Seitenkantenpaar SP₁ (vgl. Fig. 13 b). In dieser Position des Deviationskontrollschiebers 218 wäre die vom Orthesengelenk 2 zugelassene Deviationsbewegung urD in beide Richtungen bis auf einen sehr kleinen Winkelbereich, der sich durch die oben erwähnte Federwirkung ergibt, durch Wechselwirkungen der Außenkanten 2185 des Deviationskontrollschiebers 218 mit den Seitenkanten 2123 blockiert und die Hand somit fixiert. Eine vollständige Fixierung kann durch eine Modifikation insbesondere des Grundelements 213 des Orthesengelenks 2 gewährleistet werden, welche weiter unten im Zusammenhang mit Fig. 14 näher beschrieben wird.

In Fig. 13a ist darüber hinaus zu sehen, dass der Deviationskontrollschieber 218 und der Fixierschieber 219 insbesondere verschiebbar gelagert und mittels des Kontrollstifts 2169 des zweiten Stellmittels 2167 gegeneinander verschiebbar ausgebildet sein können. Das zweite Stellmittel 2167, welches beispielsweise als eine Schraube ausgebildet sein kann, welche an einer Seite den Kontrollstift 2169 und an einer gegenüberliegenden Seite eine Nut 2168 aufweist, kann mittels des Kontrollstifts 2169 sowohl mit dem Deviationsschieber 218 als auch mit dem Fixierschieber 219 wechselwirken. Dazu kann der Kontrollstift 2169 des zweiten Stellmittels 2167 vorzugsweise so positioniert sein, dass er sich sowohl in die Führungsbahn 2184 des Deviationskontrollschiebers 218 als auch in die Führungsbahn 2194 des Fixierschiebers 219 hinein erstreckt und sich in beiden Führungsbahnen 2184 und 2194 bewegen kann.

In den Figuren 13a bis c sind das zweite Stellmittel 2167 (bzw. dessen sichtbare Teile) sowie dessen Kontrollstift 2169 jeweils schraffiert dargestellt. In den Fig. 13 a und 13 c sind zudem die beiden Schieber 218 und 219 "massiv dargestellt", sodass der Deviationskontrollschieber 218 den Fixierschieber 219 in unterschiedlichem Ausmaß verdeckt. In Fig. 13 b ist der Deviationsschieber 218 dagegen als Umrisszeichnung gezeigt, sodass der Fixierschieber 219 durch den Deviationskontrollschieber 218 hindurchscheint und der Verlauf der beiden Führungsbahnen 2184 und 2194 dieser Ausgestaltung der Erfindung sichtbar ist.

Aufgrund der unterschiedlich geformten und zueinander orientierten Führungsbahnen 2184 und 2194 kann bei einer Drehung des zweiten Stellmittels 2167 nun zunächst der Deviationskontrollschieber 218 von der Endkante 2128 der ersten Aussparung 2122 weg in Richtung des Fixierschiebers 219 geschoben werden, wobei der Fixierschieber 219 vorzugsweise zunächst in seiner Position verharren kann.

Dies kann beispielsweise zu einer Einstellung des Orthesengelenks 2 führen, wie sie in Fig. 13 b gezeigt ist.

In dieser Einstellung des Orthesengelenks 2 ragt der Kopfbereich 2181 des Deviationskontrollschiebers 218 zu einem gewissen Grad in die erste Aussparung 2122 hinein, wobei kein Kontakt mehr zwischen den Außenkanten 2185 des Deviationskontrollschiebers 218 und den Seitenkanten 2123 der ersten Aussparung 2122 besteht. Das Kontrollelement 212 kann sich in dieser Einstellung um den Drehzylinder 2121 bzw. um seine durch besagten Drehzylinder 2121 verlaufende Drehachse DA drehen und der Benutzer kann eine Deviationsbewegung urD der Hand ausführen. In Fig. 13b ist eine solche Deviationsbewegung urD durch einen kleinen gebogenen Pfeil, der eine Drehung des Kontrollelements 212 im Uhrzeigersinn andeutet, exemplarisch dargestellt. Die Deviationsbewegung urD ist in dieser Einstellung allerdings nur solange möglich, bis der Kopfbereich 2181, insbesondere dessen Hebelelement 2183, wieder mit einer Seitenkante 2123 der ersten Aussparung 2122 in Kontakt kommt (angedeutet durch einen geraden gestrichelten Pfeil in Fig. 13 b). Der Mechanismus funktioniert hier wie bei den anderen, weiter oben beschriebenen Ausgestaltungen (vgl. hierzu Fig. 7a bis 7d und Fig. 8a bis 8d).

Im Moment des Kontakts zwischen Hebelelement 2183 und Seitenkante 2123 dämpft besagtes Hebelelement 2183 aufgrund seiner Federeigenschaften den Anschlag vorteilhaft ab, so dass der Benutzer das Abstoppen in den jeweiligen Endpunkten der Deviationsbewegung urD vorteilhaft als weicher empfinden kann, als bei den beiden anderen gezeigten Ausgestaltungen der vorliegenden Erfindung. In Fig. 13 b ist diese mögliche Dämpfungsbewegung am oberen der beiden Hebelelemente 2183 beispielhaft durch einen kleinen gebogenen Pfeil angedeutet.

**Fig. 13 c** zeigt schließlich eine Einstellung des Orthesengelenks 2, in der das zweite Stellmittel 2167 weitergedreht wurde, bis dessen Kontrollstift 2169 fast bis zum Ende der Führungsbahn 2184 gewandert ist. Während dieser Drehung zieht der Kontrollstift 2169 den Deviationsschieber 218 einerseits weiter aus der ersten Aussparung 2122 heraus, schiebt andererseits den Fixierschieber 219 jedoch ab ungefähr der Hälfte seines Weges vorteilhaft in Richtung der ersten Aussparung 2122, sodass schließlich eine Endposition wie in Fig. 13 c gezeigt erreicht werden kann.

Das Kopfelement 2193 des Fixierschiebers 219 kann in dieser Endposition vorteilhaft in eine der vierten Aussparungen 2120, die am Kontrollelement 212, insbesondere innerhalb dessen erster Aussparung 2122, angeordnet sind, eingreifen und das Orthesengelenk 2 auf diese Weise wiederum fixieren. Ist eine Vielzahl an vierten Aussparungen 2120 vorgesehen, wie hier beispielsweise vier über einen Winkelbereich verteilte, kann die Fixierung nicht nur in einer vergleichsweise geraden Position der Hand, wie hier dargestellt, erfolgen, sondern es kann auch vorteilhaft eine Fixierung bei einer, bezüglich einer Deviationsbewegung urD abgewinkelten Hand erfolgen, je nachdem, wie das Kontrollelement 212 zuvor bezüglich seiner Drehachse DA gedreht wurde.

**Fig. 14** zeigt schließlich eine Ansicht einer Ausgestaltung eines Grundelements 213 eines Orthesengelenks 2 wie in Fig. 10 gezeigt, sowie einen darin eingeschobenen Deviationskontrollschieber 218.

Wie bereits weiter oben erwähnt, kann aufgrund der Ausgestaltung des Kopfbereichs 2181 des Deviationskontrollschiebers 218 in dieser Ausgestaltung der Erfindung durch einfaches Heranschieben des Deviationskontrollschiebers 218 an die Endkante 2128 der ersten Aussparung 2122 keine vollständige Fixierung der Deviationsbewegung urD erreicht werden, da die Hebelelement 2183 für sich genommen stets eine gewisse Federbewegung ausführen können. Um auch bei dieser Ausgestaltung des erfindungsgemäßen Orthesengelenks 2 eine vollständige Fixierung in einer ausgetreckten Handposition zu ermöglichen, kann, wie in Fig. 14 gezeigt, am Grundelement 213 des Orthesengelenks 2 besonders bevorzugt ein Anschlagszapfen 2133 angeordnet sein. Dieser Anschlagszapfen 2133 ist dabei vorzugsweise so auf dem Grundelement 213 positioniert und fest mit diesem verbunden, dass sich besagter Anschlagzapfen 2133 bei vollständig zur Endkante 2128 hin geschobenem Deviationskontrollschieber 218 (also der in Fig. 13 a gezeigten Situation) genau zwischen den Hebelelementen 2183 befindet. Der Anschlagszapfen 2133 verhindert dann vorteilhaft eine Federbewegung der Hebelarme 2183 nach innen, also zueinander, wenn auf die Außenkanten 2185 des Deviationsschiebers 218 über die jeweiligen Seitenkanten 2123 des ersten Seitenkantenpaares SP₁ eine Krafteinwirkung erfolgt und ermöglicht so auch bei dieser Ausgestaltung vorteilhaft eine vollständige Fixierung der Hand.

Die vorliegende Erfindung betrifft eine einstellbares Orthesengelenk 2 zur kontrollierten Bewegung und/oder Fixierung einer Hand sowie eine Orthese 1 mit einem derartigen Orthesengelenk 2. Das erfindungsgemäße Orthesengelenk 2 zeichnet sich durch ein Bewegungsmodul 21 aus, welches wenigstens ein Grundelement 213 und wenigstens ein Kontrollelement 212 mit einer Oberseite 2126 und einer dem Grundelement 213 zugewandten und der Oberseite 2126 gegenüberliegenden Unterseite 2127 umfasst, wobei an der Unterseite 2127 ein Drehzylinder 2121 angeordnet ist, mittels dessen das Kontrollelement 212 gegenüber dem Grundelement 213 bezüglich einer Deviationsachse DA drehbar gelagert ist, wobei das Kontrollelement 212 eingerichtet ist, in seiner Drehbewegung kontrolliert beschränkt zu werden, und wobei das Kontrollelement 212 eingerichtet ist; die Bewegung eines, mit dem Bewegungsmodul 21 wirkverbundenen Schwenkhebels 22 in seinem Bewegungsumfang kontrolliert zu beschränken. Es kann ohne Werkzeug durch den Patienten selbst eingestellt werden und ist so kompakt gebaut, dass seine Benutzung im Alltag als nicht störend empfunden wird.

### Bezugszeichenliste

- 1: Orthese
- 11: Unterarmbefestigung
- 12: Befestigungsmittel
- 13: Gehäuse für zweiten Kontrollschieber (215)

- 2: Orthesengelenk
- 21: Bewegungsmodul
211 Deckelement
2111 Aussparung für Schwenkhebel (22)
212 Kontrollelement
2121 Drehzylinder
2122 Erste Aussparung zur Deviationskontrolle
2123 Seitenkante der Aussparung (2122)
2124 Zweite Aussparung für Schwenkhebel (22)
2125 Dritte Aussparung für ersten Kontrollschieber (214)
2126 Oberseite
2127 Unterseite
2128 Endkante der Aussparung (2122)
2129 Rand
2120 Vierte Aussparung zur Deviationsfixierung
213 Grundelement
2131 Aufnahme für Drehzylinder (2121)
2132 Führungsmittel für Drehzylinder (2121)
2133 Anschlagzapfen
214 Erster Kontrollschieber für Extension/Flexion (E/F)
2141 Aussparung für erstes Stellmittel (216)
2142 gabelförmiges Ende
2143 Verbindungsstift
215 Zweiter Kontrollschieber für ulnare/radiale Deviation (urD) der Hand
2151 Einrastmechanismus
2152 Länge
2153 Breite
2154 Höhe
2155 Bedienmittel
216 Erstes Stellmittel
2161 Kopf
2162 Nut
2163 Kragen
2164 Stellzylinder
2165 Längsachse
2166 Kanal
2167 Zweites Stellmittel
2168 Nut
2169 Kontrollstift
217 Einrastmechanismus
2171 Feder
2172 Vertiefung
218 Deviationskontrollschieber
2181 Kopfbereich
2182 Körperbereich
2183 Hebelelement des Deviationskontrollschiebers (218)
2184 Führungsbahn des Deviationskontrollschiebers (218)
2185 Außenkante der Hebelelements (2183)
219 Fixierschieber
2191 Kopfbereich des Fixierschiebers (219)
2192 Körperbereich des Fixierschiebers (219)
2193 Kopfelement des Fixierschiebers (219)
2194 Führungsbahn des Fixierschiebers (219)

- 22: Schwenkhebel für Extension/Flexion (E/F)
221 Schwenkachse

- 3: Verbindungsmittel

- 4: Handbefestigung
- 41: Befestigungsmittel

- A: Abstand der Außenkanten (2185) der Hebelelemente (2183)

- LA: Längsachse der Orthese (1)
- DA: Deviationsachse des Orthesengelenks (2)
- E/F: Extension und Flexion der Hand
- urD: ulnare und radiale Deviation der Hand
- dₙ: Abstand der Aussparungskanten (2123)
- h: Höhe des Kontrollelements (212) im Bereich der ersten Aussparung (2122)
- H: Höhe des Kontrollelements (212) außerhalb des Bereichs der Aussparung (2122)
- SPₙ: Seitenkantenpaar
- |W_{D1}|, |W_{D2}|: Betrag des Deviationswinkels
- |W_{E/F}|: Betrag des Extensions-/Flexionswinkels

## Patentansprüche

1. Einstellbares Orthesengelenk (2) zur kontrollierten Bewegung und/oder Fixierung einer Hand eines Patienten, wenigstens umfassend
- ein Bewegungsmodul (21) zur Kontrolle einer Extensions- und/oder Flexionsbewegung (E/F) sowie zur Kontrolle und Ausführung einer ulnaren und/oder radialen Deviation (urD) der Hand; und
- einen, mit dem Bewegungsmodul (21) wirkverbundenen Schwenkhebel (22) zur Ausführung der Extensions- und/oder Flexionsbewegung (E/F) der Hand;
**dadurch gekennzeichnet, dass**
- das Bewegungsmodul (21)
- wenigstens ein Grundelement (213) und
- wenigstens ein Kontrollelement (212) mit einer Oberseite (2126) und einer dem Grundelement (213) zugewandten und der Oberseite (2126) gegenüberliegenden Unterseite (2127) umfasst,
- wobei an der Unterseite (2127) ein Drehzylinder (2121) angeordnet ist, mittels dessen das Kontrollelement (212) gegenüber dem Grundelement (213) bezüglich einer Deviationsachse (DA) drehbar gelagert ist,
- wobei an der Unterseite (2127) eine erste Aussparung (2122) zur Deviationskontrolle angeordnet ist;
- wobei das Kontrollelement (212) wenigstens einen ersten Kontrollschieber (214) umfasst, welcher mittels wenigstens eines ersten Stellmittels (216) bezüglich des mit dem Bewegungsmodul (21) wirkverbundenen Schwenkhebels (22) verschiebbar ist;
- und wobei das Kontrollelement (212) eingerichtet ist; die Bewegung des Schwenkhebels (22) in seinem Bewegungsumfang mittels des ersten Kontrollschiebers (214) kontrolliert zu beschränken;
- und dass das Orthesengelenk (2) wenigstens einen, mit dem Kontrollelement (212) zusammenwirkenden zweiten Kontrollschieber (215) für die Kontrolle der ulnaren und radialen Deviationsbewegung (urD) der Hand umfasst;
- wobei das Kontrollelement (212) eingerichtet ist, in seiner Drehbewegung um die Deviationsachse (DA) durch ein Zusammenwirken der ersten Aussparung (2122) zur Deviationskontrolle und des wenigstens einen zweiten Kontrollschiebers (215) kontrolliert beschränkt zu werden.

2. Orthesengelenk (2) nach Anspruch 1, **dadurch gekennzeichnet, dass** der erste Kontrollschieber (214) im Wesentlichen quaderförmig ausgestaltet und an seinem, dem Schwenkhebel (22) zugewandten Ende gabelförmig ausgebildet ist.

3. Orthesengelenk (2) nach Anspruch 2, **dadurch gekennzeichnet, dass** das erste Stellmittel (216) wenigstens einen Kopf (2161) und einen Stellzylinder (2164) umfasst, welche, vorzugsweise über einen Kragen (2163), miteinander verbunden sind, sodass sich ein Bauteil mit einer Längsachse (2165) bildet,
- wobei der Stellzylinder (2164) einen kreisförmigen Querschnitt aufweist und bezüglich der Längsachse (2165) außermittig mit dem Kopf (2161) des ersten Stellmittels (216) verbunden ist.

4. Orthesengelenk (2) nach Anspruch 3, **dadurch gekennzeichnet, dass** der erste Kontrollschieber (214) wenigstens eine Aussparung (2141) umfasst, welche eingerichtet ist, den Stellzylinder (2164) aufzunehmen und mit diesem wechselzuwirken.

5. Orthesengelenk (2) nach Anspruch 2, **dadurch gekennzeichnet, dass** das erste Stellmittel (216) wenigstens einen Kopf (2161) und einen Stellzylinder (2164) umfasst, welche, vorzugsweise über einen Kragen (2163), miteinander verbunden sind, sodass sich ein Bauteil mit einer Längsachse (2165) bildet,
- wobei der Stellzylinder (2164) an seiner dem Kopf (2161) abgewandten Seite einen, vorzugsweise im Wesentlichen C-förmigen, Kanal (2166) aufweist.

6. Orthesengelenk (2) nach Anspruch 5, **dadurch gekennzeichnet, dass** der erste Kontrollschieber (214) einen Verbindungsstift (2143) umfasst, welcher bei Gebrauch des Orthesengelenks (2) in den Kanal (2166) des Stellzylinders (2164) eingreift,
- wobei der Verbindungsstift (2143) vorzugsweise innerhalb einer Aussparung (2141) des ersten Kontrollschiebers (214) angeordnet ist, welche eingerichtet ist, den Stellzylinder (2164) aufzunehmen.

7. Orthesengelenk (2) nach einem oder mehreren der Ansprüche 2 bis 6, **dadurch gekennzeichnet, dass** das erste Stellmittel (216) und/oder der erste Kontrollschieber (214) wenigstens einen Einrastmechanismus (217) umfasst, welcher eingerichtet ist, den ersten Kontrollschieber (214) in seiner Verschiebbewegung bezüglich des mit dem Bewegungsmodul (21) wirkverbundenen Schwenkhebel (22) an wenigstens einer vorgegebenen Position reversibel zu fixieren.

8. Orthesengelenk (2) nach einem oder mehreren der vorherigen Ansprüche, **dadurch gekennzeichnet, dass** sich die erste Aussparung (2122) zur Deviationskontrolle
- von einem Rand (2129) des Kontrollelements (212) in Richtung des Drehzylinders (2121) erstreckt, sodass eine Höhe (h) des Kontrollelements (212) im Bereich der ersten Aussparung (2122) kleiner ist als eine Höhe (H) des Kontrollelements (212) im Bereich außerhalb der ersten Aussparung (2122),
- wodurch sich wenigstens vier Seitenkanten (2123) bilden, die den Bereich innerhalb der ersten Aussparung (2122) vom Bereich außerhalb der ersten Aussparung (2122) abgrenzen,
- von denen je zwei in einem Abstand dₙ mit n = 1, 2, ... einander im Wesentlichen gegenüberliegen und ein Seitenkantenpaar SPₙ mit n = 1, 2, ... bilden;
- wobei ein Abstand dₙ zwischen zwei näher am Drehzylinder (2121) angeordneten, einander gegenüberliegenden Seitenkanten (2123) eines Seitenkantenpaares SPₙ stets kleiner ist als ein Abstand dₙ₊₁ zwischen zwei näher am Rand (2129) des Kontrollelements (212) angeordneten, einander gegenüberliegenden Seitenkanten (2123) eines Seitenkantenpaares SPₙ₊₁.

9. Orthesengelenk (2) nach Anspruch 8, **dadurch gekennzeichnet, dass** eine Vielzahl von Seitenkanten (2123), vorzugsweise acht Seitenkanten (2123), gebildet werden, von denen je zwei einander im Wesentlichen gegenüberliegen und ein Seitenkantenpaar SPₙ mit n = 1, 2, ... bilden.

10. Orthesengelenk (2) nach Anspruch 8 oder 9, **dadurch gekennzeichnet, dass** der zweite Kontrollschieber (215) im Wesentlichen quaderförmig ausgebildet ist und eine Länge (2152), eine Breite (2153) und eine Höhe (2154) aufweist;
- wobei die Breite (2153) dem Abstand d₁ der einander gegenüberliegenden Seitenkanten (2123) des ersten, am nächsten zum Drehzylinder (2121) angeordneten Seitenkantenpaares SP₁ entspricht;
- und wobei der zweite Kontrollschieber (215) in die erste Aussparung (2122) des Kontrollelements (212) hineinführbar und herausführbar ausgestaltet ist und mit den Seitenkanten (2123) wechselwirken kann.

11. Orthesengelenk (2) nach Anspruch 8 oder 9, **gekennzeichnet durch** zwei, mit dem Kontrollelement (212) zusammenwirkende zweite Kontrollschieber (215) für die Kontrolle der ulnaren/radialen Deviationsbewegung (urD) der Hand,
- wobei die zweiten Kontrollschieber (215) jeweils im Wesentlichen quaderförmig ausgebildet sind und eine Länge (2152), eine Breite (2153) und eine Höhe (2154) aufweisen;
- wobei die Summe der Breiten (2153) dem Abstand d₁ der einander gegenüberliegenden Seitenkanten (2123) des ersten, am nächsten zum Drehzylinder (2121) angeordneten Seitenkantenpaares SP₁ entspricht;
- und wobei ein jeder zweite Kontrollschieber (215) in die erste Aussparung (2122) des Kontrollelements (212) hineinführbar und herausführbar ausgestaltet ist und mit den Seitenkanten (2123) wechselwirken kann.

12. Orthesengelenk (2) nach Anspruch 10 oder 11, **dadurch gekennzeichnet, dass** ein jeder zweite Kontrollschieber (215) eingerichtet ist, an vorgegebenen Positionen während des Hineinführens in und Herausführens aus der ersten Aussparung (2122) reversibel fixiert zu werden, vorzugsweise mittels eines Einrastmechanismus (2151).

13. Orthesengelenk (2) nach Anspruch 8 oder 9, **gekennzeichnet durch** zwei, mit dem Kontrollelement (212) zusammenwirkende zweite Kontrollschieber (215) für die Kontrolle der ulnaren/radialen Deviationsbewegung (urD) der Hand und wenigstens eine, am Kontrollelement (212) angeordnete, vierte Aussparung (2120) zur Deviationsfixierung;
- wobei einer der zweiten Kontrollschieber (215) als Deviationskontrollschieber (218) ausgebildet ist, wenigstens umfassend:
- einen Kopfbereich (2181), welcher eingerichtet ist in die erste Aussparung (2122) des Kontrollelements (212) hineingeführt und herausgeführt zu werden und mit den Seitenkanten (2123) der ersten Aussparung (2122) des Kontrollelements (212) wechselzuwirken,
- und einen Körperbereich (2182), welcher eine Führungsbahn (2184) für einen Kontrollstift (2169) eines, vom Orthesengelenk (2) umfassten, zweiten Stellmittels (2167) aufweist;
- wobei der andere der zwei zweiten Kontrollschieber (215) als ein Fixierschieber (219) ausgebildet ist, wenigstens umfassend:
- einen Kopfbereich (2191), welcher eingerichtet ist mit der vierten Aussparung (2120) des Kontrollelement (212) wechselzuwirken,
- und einen Körperbereich (2192), welcher eine Führungsbahn (2194) für den Kontrollstift (2169) des zweiten Stellmittels (2167) aufweist;
- und wobei der Deviationskontrollschieber (218) und der Fixierschieber (219) verschiebbar gelagert und mittels des Kontrollstifts (2169) des zweiten Stellmittels (2167) gegeneinander verschiebbar ausgebildet sind.

14. Orthesengelenk (2) nach Anspruch 13, **dadurch gekennzeichnet,**
- **dass** der Kopfbereich (2181) des Deviationskontrollschiebers (218) von zwei, einander gegenüberliegenden Hebelelementen (2183) gebildet wird, welche gegeneinander federnd bewegbar sind und deren Außenkanten (2185) in einem entspannten Zustand zueinander einen Abstand (A) aufweisen, der dem Abstand d₁ der einander gegenüberliegenden Seitenkanten (2123) des ersten, am nächsten zum Drehzylinder (2121) angeordneten Seitenkantenpaares SP₁ der ersten Aussparung (2122) entspricht;
- und **dass** der Kopfbereich (2191) des Fixierschiebers (219) ein Kopfelement (2193) zur Wechselwirkung mit der vierten Aussparung (2120) umfasst, welches fest mit dem Körperbereich (2192) des Fixierschiebers (219) verbunden ist.

15. Orthese (1) zur kontrollierten Bewegung und/oder Fixierung einer Hand oder einer Hand und eines Unterarms eines Patienten, **gekennzeichnet durch** ein einstellbares Orthesengelenk (2) nach einem oder mehreren der vorhergehenden Ansprüche 1 bis 14.

## Claims

1. An adjustable orthosis joint (2) for the controlled movement and/or fixation of a patient's hand, at least comprising
- a movement module (21) for controlling an extension and/or flexion movement (E/F) and for controlling and executing an ulnar and/or radial deviation (urD) of the hand; and
- a pivot lever (22), which is operatively connected to the movement module (21), for carrying out the extension and/or flexion movement (E/F) of the hand;
**characterized in that**
- the movement module (21) comprises
- at least one base element (213) and
- at least one control element (212) with a top side (2126) and a bottom side (2127) facing the base element (213) and lying opposite the top side (2126),
- wherein a rotary cylinder (2121), by way of which the control element (212) is mounted rotatably relative to the base element (213) with respect to a deviation axis (DA), is arranged on the bottom side (2127),
- wherein a first cutout (2122) is arranged on the bottom side (2127) for deviation control;
- wherein the control element (212) comprises at least one first control slide (214), which can be moved by means of at least one first actuating means (216) with respect to the pivot lever (22) which is operatively connected to the movement module (21);
- and wherein the control element (212) is designed to restrict the range of movement of the pivot lever (22) by the first control slide (214) in a controlled manner;
- and wherein the orthosis joint (2) comprises at least a second control slide (215) which interacts with the control element (212) to control the ulnar and radial deviation movement (urD) of the hand;
- wherein the control element (212) is designed such that its rotational movement around the deviation axis (DA) can be restricted in a controlled manner by an interaction of the first cutout (2122) of the deviation control and the at least second control slide (215).

2. The orthosis joint (2) as claimed in claim 1, **characterized in that** the first control slide (214) is of substantially cuboid-shaped configuration and, at its end facing the pivot lever (22), is of fork-shaped configuration.

3. The orthosis joint (2) as claimed in claim 2, **characterized in that** the first actuating means (216) comprises at least one head (2161) and one actuating cylinder (2164) which are connected to each other, preferably via a collar (2163), with the result that a component with a longitudinal axis (2165) is formed,
- wherein the actuating cylinder (2164) has a circular cross section and is connected to the head (2161) of the first actuating means (216) eccentrically with respect to the longitudinal axis (2165).

4. The orthosis joint (2) as claimed in claim 3, **characterized in that** the first control slide (214) comprises at least one cutout (2141) which is configured to receive the actuating cylinder (2164) and to interact with it.

5. The orthosis joint (2) as claimed in claim 2, **characterized in that** the first actuating means (216) comprises at least one head (2161) and one actuating cylinder (2164) which are connected to each other, preferably via a collar (2163), with the result that a component with a longitudinal axis (2165) is formed,
- wherein the actuating cylinder (2164), on its side facing away from the head (2161), has a, preferably substantially C-shaped, channel (2166).

6. The orthosis joint (2) as claimed in claim 5, **characterized in that** the first control slide (214) comprises a connecting pin (2143) which engages in the channel (2166) of the actuating cylinder (2164) during use of the orthosis joint (2),
- wherein the connecting pin (2143) is preferably arranged within a cutout (2141) of the first control slide (214), which is configured to receive the actuating cylinder (2164).

7. The orthosis joint (2) as claimed in one or more of claims 2 to 6, **characterized in that** the first actuating means (216) and/or the first control slide (214) comprises at least one snap-in mechanism (217) which is configured to reversibly fix the first control slide (214) in its displacement movement with respect to the pivot lever (22) which is operatively connected to the movement module (21) at at least one predetermined position.

8. The orthosis joint (2) as claimed in one or more of the preceding claims, **characterized in that** the first cutout (2122) for deviation control,
- extends from an edge (2129) of the control element (212) in the direction of the rotary cylinder (2121), with the result that a height (h) of the control element (212) in the region of the first cutout (2122) is smaller than a height (H) of the control element (212) in the region outside the first cutout (2122),
- as a result of which at least four side edges (2123) are formed which delimit the region within the first cutout (2122) from the area outside the first cutout (2122),
- of which two lie substantially opposite each other at a spacing dₙ where n = 1, 2, ... and form a side edge pair SPₙ where n = 1, 2, ...;
- wherein a spacing dₙ between two side edges (2123), which are arranged closer to the rotary cylinder (2121) and lie opposite one another, of a side edge pair SPₙ is always smaller than a spacing dₙ₊₁ between two side edges (2123), which are arranged closer to the edge (2129) of the control element (212) and lie opposite one another, of a side edge pair SPₙ₊₁.

9. The orthosis joint (2) as claimed in claim 8, **characterized in that** a multiplicity of side edges (2123), preferably eight side edges (2123), are formed, of which two each lie substantially opposite each other and form a side edge pair SPₙ where n = 1, 2, ....

10. The orthosis joint (2) as claimed in claim 8 or 9, **characterized in that** the second control slide (215) is of substantially cuboid-shaped configuration and has a length (2152), a width (2153) and a height (2154);
- wherein the width (2153) corresponds to the spacing d₁ of the opposite side edges (2123) of the first side edge pair SP₁ arranged closest to the rotary cylinder (2121);
- and wherein the second control slide (215) is configured such that it can be inserted into the first cutout (2122) of the control element (212) and can be removed from it and can interact with the side edges (2123).

11. The orthosis joint (2) as claimed in claim 8 or 9, **characterized by** two second control slides (215) which interact with the control element (212) for the control of the ulnar/radial deviation movement (urD) of the hand,
- wherein the second control slides (215) are each of substantially cuboid-shaped configuration and have a length (2152), a width (2153) and a height (2154);
- wherein the sum of the widths (2153) corresponds to the spacing d₁ of the opposite side edges (2123) of the first pair of side edges SP₁ arranged closest to the rotary cylinder (2121);
- and wherein each second control slide (215) is configured such that it can be inserted into the first cutout (2122) of the control element (212) and can be removed from it and can interact with the side edges (2123).

12. The orthosis joint (2) as claimed in claim 10 or 11, **characterized in that** each second control slide (215) is configured to be reversibly fixed at predetermined positions during insertion into and removal from the first cutout (2122), preferably by means of a snap-in mechanism (2151).

13. The orthosis joint (2) as claimed in claim 8 or 9, **characterized by** two second control slides (215) which interact with the control element (212) for the control of the ulnar/radial deviation movement (urD) of the hand and at least one fourth cutout (2120) for the deviation fixation which is arranged on the control element (212);
- wherein one of the second control slides (215) is designed as a deviation control slide (218), at least comprising:
- a head region (2181) which is configured to be inserted into the first cutout (2122) of the control element (212) and to be removed from it, and to interact with the side edges (2123) of the first cutout (2122) of the control element (212),
- and a body region (2182) which has a guide track (2184) for a control pin (2169) of a second actuating means (2167) which is covered by the orthosis joint (2);
- wherein the other of the two second control slides (215) is designed as a fixing slide (219), at least comprising:
- a head region (2191) which is configured to interact with the fourth cutout (2120) of the control element (212),
- and a body region (2192) which has a guide track (2194) for the control pin (2169) of the second actuating means (2167);
- and wherein the deviation control slide (218) and the fixing slide (219) are displaceably mounted and configured by means of the control pin (2169) of the second actuating means (2167) in a displaceable manner with respect to each other.

14. The orthosis joint (2) as claimed in claim 13, **characterized**
- **in that** the head region (2181) of the deviation control slide (218) is formed by two opposite lever elements (2183) which are resiliently movable with respect to each other and the outer edges (2185) of which are at a spacing (A) from each other in a relaxed state, which spacing corresponds to the spacing d₁ of the opposite side edges (2123) of the first side edge pair SP₁, arranged closest to the rotary cylinder (2121), of the first cutout (2122);
- and **in that** the head region (2191) of the fixing slide (219) comprises a head element (2193) for interaction with the fourth cutout (2120), which is firmly connected to the body region (2192) of the fixing slide (219).

15. An orthosis (1) for the controlled movement and/or fixation of a hand or a hand and a forearm of a patient, **characterized by** an adjustable orthosis joint (2) as claimed in one or more of the preceding claims 1 to 14.

## Revendications

1. Articulation orthétique réglable (2) pour le mouvement contrôlé et/ou la fixation d'une main d'un patient, comprenant au moins
- un module de mouvement (21) pour contrôler un mouvement d'extension et/ou de flexion (E/F) ainsi que pour contrôler et exécuter une déviation ulnaire et/ou radiale (urD) de la main ; et
- un levier pivotant (22) relié de manière fonctionnelle au module de mouvement (21) pour exécuter le mouvement d'extension et/ou de flexion (E/F) de la main ;
**caractérisé en ce que**
- le module de mouvement (21)
- au moins un élément de base (213) et
- au moins un élément de contrôle (212) avec une face supérieure (2126) et une face inférieure (2127) tournée vers l'élément de base (213) et opposée à la face supérieure (2126),
- un cylindre rotatif (2121) étant disposé sur la face inférieure (2127), au moyen duquel l'élément de contrôle (212) est monté de manière rotative par rapport à l'élément de base (213) autour d'un axe de déviation (DA),
- un premier évidement (2122) pour le contrôle de la déviation étant disposé sur la face inférieure (2127) ;
- l'élément de contrôle (212) comprenant au moins un premier curseur de commande (214) qui peut être déplacé au moyen d'au moins un premier moyen de réglage (216) par rapport au levier pivotant (22) relié de manière fonctionnelle au module de mouvement (21) ;
- et l'élément de contrôle (212) étant conçu pour limiter de manière contrôlée le mouvement du levier pivotant (22) dans son amplitude de mouvement à l'aide du premier curseur de contrôle (214) ;
- et que l'articulation orthétique (2) comprend au moins un deuxième curseur de contrôle (215) coopérant avec l'élément de contrôle (212) pour contrôler le mouvement de déviation ulnaire et radiale (urD) de la main ;
- l'élément de contrôle (212) étant conçu pour être limité de manière contrôlée dans son mouvement de rotation autour de l'axe de déviation (DA) par une interaction entre le premier évidement (2122) pour le contrôle de la déviation et le au moins un deuxième curseur de contrôle (215).

2. Articulation orthétique (2) selon la revendication 1, **caractérisée en ce que** le premier curseur de contrôle (214) est essentiellement de forme parallélépipédique et est conçu en forme de fourche à son extrémité tournée vers le levier pivotant (22).

3. Articulation orthétique (2) selon la revendication 2, **caractérisée en ce que** le premier moyen de réglage (216) comprend au moins une tête (2161) et un vérin de réglage (2164) qui sont reliés entre eux, de préférence par une collerette (2163), de manière à former un élément avec un axe longitudinal (2165)
- le vérin de réglage (2164) présentant une section transversale circulaire et étant relié de manière excentrée par rapport à l'axe longitudinal (2165) à la tête (2161) du premier moyen de réglage (216).

4. Articulation orthétique (2) selon la revendication 3, **caractérisée en ce que** le premier curseur de contrôle (214) comprend au moins un évidement (2141) qui est agencé pour recevoir le vérin de réglage (2164) et pour interagir avec celui-ci.

5. Articulation orthétique (2) selon la revendication 2, **caractérisée en ce que** le premier moyen de réglage (216) comprend au moins une tête (2161) et un vérin de réglage (2164) qui sont reliés entre eux, de préférence par une collerette (2163), de manière à former un élément avec un axe longitudinal (2165),
- le vérin de réglage (2164) présentant, sur son côté opposé à la tête (2161), un canal (2166) de préférence essentiellement en forme de C.

6. Articulation orthétique (2) selon la revendication 5, **caractérisée en ce que** le premier curseur de contrôle (214) comprend une goupille de liaison (2143) qui, lors de l'utilisation de l'articulation orthétique (2), s'engage dans le canal (2166) du vérin de réglage (2164),
- la tige de liaison (2143) étant de préférence disposée à l'intérieur d'un évidement (2141) du premier curseur de commande (214) qui est conçu pour recevoir le vérin de réglage (2164).

7. Articulation orthétique (2) selon une ou plusieurs des revendications 2 à 6, **caractérisée en ce que** le premier moyen de réglage (216) et/ou le premier curseur de contrôle (214) comprend au moins un mécanisme d'encliquetage (217) qui est conçu pour fixer de manière réversible le premier curseur de commande (214) dans son mouvement de déplacement par rapport au levier pivotant (22) relié de manière fonctionnelle au module de mouvement (21) dans au moins une position prédéfinie.

8. Articulation orthétique (2) selon une ou plusieurs des revendications précédentes, **caractérisée en ce que** le premier évidement (2122) pour le contrôle de la déviation
- s'étend depuis un bord (2129) de l'élément de contrôle (212) en direction du cylindre rotatif (2121), de sorte qu'une hauteur (h) de l'élément de contrôle (212) dans la zone du premier évidement (2122) est inférieur à une hauteur (H) de l'élément de contrôle (212) dans la zone à l'extérieur du premier évidement (2122),
- ce qui forme au moins quatre arêtes latérales (2123) qui délimitent la zone à l'intérieur du premier évidement (2122) de la zone à l'extérieur du premier évidement (2122),
- dont deux sont sensiblement opposés l'un à l'autre à une distance dₙ avec n = 1, 2, ... et forment une paire de bords latéraux SPₙ avec n = 1, 2, ...;
- la distance dₙ entre deux bords latéraux (2123) opposés d'une paire de bords latéraux SPₙ situés plus près du cylindre rotatif (2121) étant toujours inférieure à la distance dₙ₊₁ entre deux bords latéraux (2123) opposés d'une paire de bords latéraux SPₙ₊₁ disposés plus près du bord (2129) de l'élément de contrôle (212).

9. Articulation orthétique (2) selon la revendication 8, **caractérisée en ce qu'**une pluralité de bords latéraux (2123), de préférence huit bords latéraux (2123), sont formés, dont deux sont essentiellement opposés l'un à l'autre et forment une paire de bords latéraux SPₙ avec n = 1, 2, ...

10. Articulation orthétique (2) selon la revendication 8 ou 9, **caractérisée en ce que** le deuxième curseur de contrôle (215) est essentiellement de forme parallélépipédique et présente une longueur (2152), une largeur (2153) et une hauteur (2154) ;
- la largeur (2153) correspondant à la distance d₁ entre les arêtes latérales opposées (2123) de la première paire d'arêtes latérales SP₁ disposée le plus près du cylindre rotatif (2121) ;
- et le deuxième curseur de contrôle (215) étant conçu de manière à pouvoir être introduit dans le premier évidement (2122) de l'élément de contrôle (212) et en être retiré, et pouvant interagir avec les bords latéraux (2123).

11. Articulation orthétique (2) selon la revendication 8 ou 9, **caractérisée par** deux deuxièmes curseurs de contrôle (215) coopérant avec l'élément de contrôle (212) pour contrôler le mouvement de déviation ulnaire/radiale (urD) de la main,
- les deuxièmes curseurs de contrôle (215) étant chacun essentiellement de forme parallélépipédique et présentant une longueur (2152), une largeur (2153) et une hauteur (2154) ;
- la somme des largeurs (2153) correspondant à la distance d₁ entre les bords latéraux opposés (2123) de la première paire de bords latéraux SP₁ la plus proche du cylindre rotatif (2121) ;
- et chaque deuxième curseur de contrôle (215) étant conçu de manière à pouvoir être introduit dans le premier évidement (2122) de l'élément de contrôle (212) et en être retiré, et pouvant interagir avec les bords latéraux (2123).

12. Articulation orthétique (2) selon la revendication 10 ou 11, **caractérisée en ce que** chaque deuxième curseur de contrôle (215) est conçu pour être fixé de manière réversible à des positions prédéfinies pendant son insertion dans et son retrait du premier évidement (2122), de préférence au moyen d'un mécanisme d'encliquetage (2151).

13. Articulation orthétique (2) selon la revendication 8 ou 9, **caractérisée par** deux deuxièmes curseurs de contrôle (215) coopérant avec l'élément de contrôle (212) pour le contrôle du mouvement de déviation ulnaire/radiale (urD) de la main et au moins un quatrième évidement (2120) disposé sur l'élément de contrôle (212) pour la fixation de la déviation radial (urD) de la main et au moins un quatrième évidement (2120) pour la fixation de la déviation disposé sur l'élément de contrôle (212) ;
- l'un des seconds curseurs de contrôle (215) étant conçu comme un curseur de contrôle de déviation (218), comprenant au moins :
- une partie de tête (2181) qui est conçue pour être introduite dans le premier évidement (2122) de l'élément de contrôle (212) et pour en être retirée, et pour interagir avec les bords latéraux (2123) du premier évidement (2122) de l'élément de contrôle (212),
- et une partie centrale (2182) qui présente une glissière de guidage (2184) pour une tige de commande (2169) d'un deuxième moyen de réglage (2167) compris dans l'articulation orthopédique (2) ;
- l'autre des deux deuxièmes curseurs de contrôle (215) étant conçu comme un coulisseau de fixation (219), comprenant au moins :
- une partie de tête (2191) qui est agencée pour interagir avec le quatrième évidement (2120) de l'élément de contrôle (212),
- et une partie corps (2192) qui présente une glissière de guidage (2194) pour la tige de commande (2169) du deuxième moyen de réglage (2167);
- et dans lequel le curseur de contrôle de déviation (218) et le curseur de fixation (219) sont montés de manière coulissante et sont conçus pour pouvoir coulisser l'un par rapport à l'autre au moyen de la tige de contrôle (2169) du deuxième moyen de réglage (2167).

14. Articulation orthétique (2) selon la revendication 13, **caractérisée en ce que**
- que la partie de tête (2181) du coulisseau de contrôle de déviation (218) est formée par deux éléments de levier (2183) opposés l'un à l'autre, qui peuvent se déplacer de manière élastique l'un par rapport à l'autre et dont les bords extérieurs (2185) présentent, à l'état détendu, un écartement (A) qui correspond à l'écartement d₁ des bords latéraux opposés (2123) de la première paire de bords latéraux SP₁ du premier évidement (2122) disposée le plus près du cylindre rotatif (2121) ;
- et **en ce que** la partie de tête (2191) du coulisseau de fixation (219) comprend un élément de tête (2193) destiné à interagir avec le quatrième évidement (2120), lequel est relié de manière fixe à la zone de corps (2192) du coulisseau de fixation (219).

15. Orthèse (1) pour le mouvement contrôlé et/ou la fixation d'une main ou d'une main et d'un avant-bras d'un patient, **caractérisée par** une articulation orthétique réglable (2) selon une ou plusieurs des revendications 1 à 14 précédentes.
